# EUROPEAN PATENT SPECIFICATION

(11) **EP 1 272 160 B1**
(45) Date of publication and mention of the grant of the patent: **17.01.2007**
(21) Application number: 01921261.2
(22) Date of filing: 11.04.2001
(51) Int. Cl.: A61K 9/127, A61K 8/00, A61P 17/00

(54) **LIPID-BASED DRUG DELIVERY SYSTEMS FOR TOPICAL APPLICATION**
AUF LIPIDEN BASIERENDE ARZNEISTOFFABGABESYSTEME ZUR TOPISCHEN ANWENDUNG
SYSTEMES D'ADMINISTRATION DE MEDICAMENTS A BASE DE LIPIDES CONTENANT DES DERIVES LIPIDIQUES DEGRADABLES DE PHOSPHOLIPASE A2 DESTINES A L'APPLICATION TOPIQUE A LA PEAU

(30) Priority: 12.04.2000 DK 200000616
(43) Date of publication of application: 08.01.2003
(73) Proprietor: Liplasome Pharma A/S, 2800 Lyngby (DK)
(72) Inventor: JORGENSEN, Kent, DK-2880 Bagsvaerd (DK); DAVIDSEN, Jesper, DK-2100 Copenhagen (DK); VERMEHREN, Charlotte, DK-4100 Sotofte (DK); FROKJAER, Sven, DK-2840 Holte (DK); MOURITSEN, Ole, G., DK-5230 Odense (DK)
(74) Representative: Klinge, Ulla Callesen
(86) International application number: PCT/DK2001/000267
(87) International publication number: WO 2001/076555

(56) References cited:
- EP-A- 0 475 160
- FR-A- 2 638 639
- US-A- 5 827 836
- US-A- 5 942 246

## Description

### FIELD OF THE INVENTION

The invention relates to lipid-based pharmaceutical or cosmetic compositions for use in the treatment, prevention or alleviation of diseases or conditions in skin or mucous membranes of mammals that are associated with or resulting from increased levels of extracellular phospholipase A₂ (PLA₂) activity in the tissue.

### BACKGROUND OF THE INVENTION

The skin as an organ is of interest from biological, medical, and cosmetological points of view. There are a large number of skin diseases that are either organ-specific, e.g. psoriasis and eczemas, or are manifestations of general diseases, such as general allergic reactions. A number of these diseases confined to the skin or mucous membranes of humans or animals could be cured or alleviated by a local topical application of pharmaceutical formulations provided the application resulted in uptake of the active component. Many formulations are applied topically to the skin in order to alter the subject's appearance, to protect the subject from the environment, or to produce a biological change in the skin or other tissue for therapeutic, preventive or cosmetic purposes. However, the skin forms one of the body's most effective barriers to foreign substances and owing to skin impermeability, for example, many therapeutic agents must be applied *per os* or parenterally even though the skin is the target organ.

There are many reports on different attempts to increase the permeability of intact skin by suitable manipulations (Karzel K., Liedtke, R. K. (1989) Arzneim. Forsch./Drug Res. 39, 1487-1491). For instance: jet injection (Siddiqui & Chien (1987) Crit. Rev. Ther. Drug. Carrier. Syst. 3, 195-208.), the use of electric fields (Burnette & Ongpipattanakul (1987) J. Pharm. Sci. 76, 765-773) or chemical penetration enhancers, such as solvents and surfactants, are particularly worth mentioning. A long list of additives which have been used to enhance the penetrance of a water-soluble agent into skin, is given in the work by Aungst et al. (1986, Int. J. Pharm. 33, 225-234). This list encompasses nonionic substances (including long-chain alcohols, surfactants, zwitterionic phospholipids, etc.), anionics (most notably fatty acids), cationic long-chain amines, sulfoxides as well as different amino-derivatives; amphotheric glycinates and betaines are also mentioned. Despite all this, the problem of agent penetration into skin has as yet not at all - or not satisfactorily - been solved.

Liposomal formulations have been the focus of extensive investigation as the mode of delivery for many drugs. There is growing evidence that for topical administration, liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin. Compounds including analgesics, antibodies, hormones and high-molecular weight DNAs have been administered to the skin. Although the majority of applications resulted in the targeting of the upper epidermis (US Patent 6.180.353), the use of lipoidal carriers, liposomes, still seem to be one very promising method to increase the permeability of the skin (Egbaria & Weiner, Adv. Drug Delivery Rev. 5, 287 (1990)).

Interestingly, a number of examples of pharmaceutically active lipid-components or lipid-derivatives have been described in the art.

US 5,827,836 discloses retinoyl substituted glycerophophoethanolamines. It is stated that the compounds and salts thereof exhibit antitumor, anti-psoriatic and anti-inflammatory activities. A possible class of compounds has a fatty ether substituent in the 1-position, a retinoid ester (retinoyl) substituent in the 2-position and a phosphoethanolamine substituent in the 3-position. It is mentioned that some of the compounds can be presented in a liposome formulation.

US 4,372,949 discloses a carcinostatic and immunostimulating agent containing a lysophospholipid and a phospholipid. Examples of compounds are 3-phosphorylcholine having a C₅₋₂₂-acyloxy or C₅₋₂₂-alkoxy substituent in the 1-position, and a hydrogen, hydroxy, C₁₋₅-acyloxy or C₁₋₅-alkoxy substituent in the 2-position. It is mentioned that the agents can be dispersed in the form of micelles or lipid vesicles.

US 5,484,911 discloses nucleoside 5'-diphosphate conjugates of ether lipids which exhibit antiviral activity. The compounds may have a fatty ether/thioether substituent in the sn-1-position and a fatty acid ester substituent in the sn-2-position. The compounds are designed so as to penetrate the cell membrane whereafter the nucleoside drug is liberated by cleavage by intracellular phosphatases. It is furthermore suggested that the also liberated ether lipids may be subsequently cleaved by intracelluar PLA₂. It is suggested that the conjugates can be presented in the form of micelles which more easily can be taken up by macrophages.

US 4,622,392 discloses cytotoxic compounds of the nucleotide-lipid conjugate type.

Xia and Hui discloses the chemical synthesis of a series of ether phospholipids from D-mannitol and their properties as tumor-cytotoxic agents.

US 5,985,854, US 6,077,837, US 6,136,796 and US 6,166,089 describe prodrugs with enhanced penetration into cells, which are particular useful for treating a condition or disease in a human related to supranormal intracellular enzyme activity. The prodrugs may be sn-2-esters of lysophospholipids. Such drugs are designed so as to be cleaved by intracelluar PLA₂.

Cevc et al. (Biochem Biophys Acta (1998) 1368, p 201-215; US Patent 6.165.500) describes one formulation of liposomes (Transfersomes^{®}) which results in very flexible liposome particles which - because of their flexibility - have the ability to penetrate even through intact mammalian skin.

### BRIEF DESCRIPTION OF THE INVENTION

The present invention is directed to drug delivery systems which are particularly useful in the treatment or alleviation of diseases or conditions in skin or mucous membranes of mammals that are associated with increased levels of extracellular phospholipase A₂ (phosphatidylcholine 2-acylhydrolase) (PLA₂) activity in the tissue.

Thus, the present invention relates to the use of lipid-based drug delivery system in the form of a liposome for administration of an active drug substance selected from lysolipid derivatives, said system comprising an edge active compound, and said active drug substance is present in the lipid-based system in the form of a prodrug, said prodrug being a lipid derivative having (a) an aliphatic group of a length of at least 7 carbon atoms and an organic radical having at least 7 carbon atoms, and (b) a hydrophilic moiety, said prodrug furthermore being a substrate for extracellular PLA₂ to the extent that the organic radical can be hydrolytically cleaved off, whereas the aliphatic group remains substantially unaffected, whereby the active drug substance is liberated in the form of a lysolipid derivative which is not a substrate for lysophospholipase, for the preparation of a medicament for treatment or prevention of treatment of diseases or conditions associated with a localised increase in extracellular PLA₂ activity in cutaneous or subcutaneous tissue of a mammal.

The present invention also relates to the use of a lipid based drug delivery system in the form of a liposome for administration of an second drug substance, said system comprising an edge active compound, and said second drug substance is incorporated in the system, said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular PLA₂ to the extent that the organic radical can be hydrolytically cleaved off, whereas the aliphatic group remains substantially unaffected, so as to result in an organic acid fragment or an organic alcohol fragment and a lysolipid fragment, said lysolipid fragment not being a substrate for lysophospholipase, for the preparation of a medicament for treatment or prevention of treatment of diseases or conditions associated with a localised increase in extracellular PLA₂ activity in cutaneous or subcutaneous tissue of a mammal.

### DESCRIPTION OF THE DRAWINGS

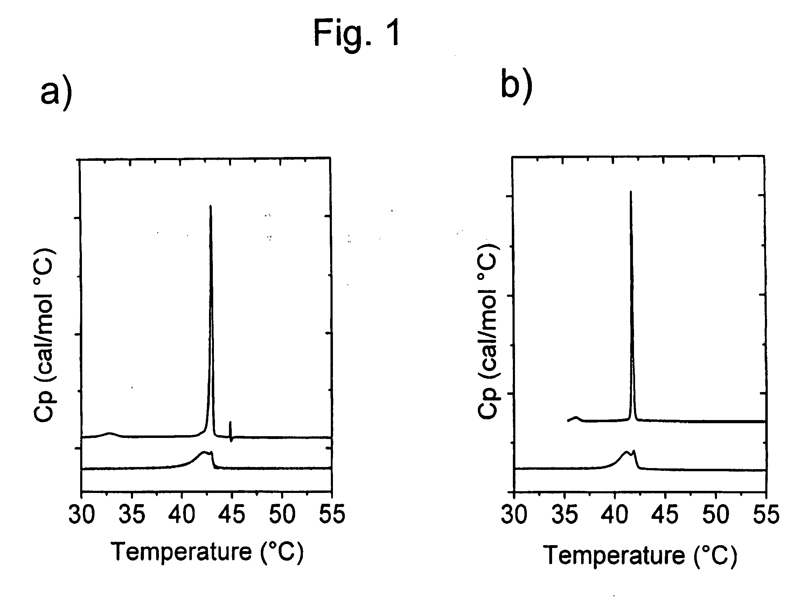

Fig. 1. Heat capacity curves obtained using differential scanning calorimetry. (a) Multilamellar, MLV (the upper curve) and unilamellar, LUV (the bottom curve) liposomes made of 1 mM 1-O-hexadecyl-2-hexadecanoyl-*sn*-glycero-3-phosphocholine (1-O-DPPC). (b) MLV (the upper curve) and LUV (the bottom curve) liposomes made of dipalmitoylphosphatidylcholine (DPPC).

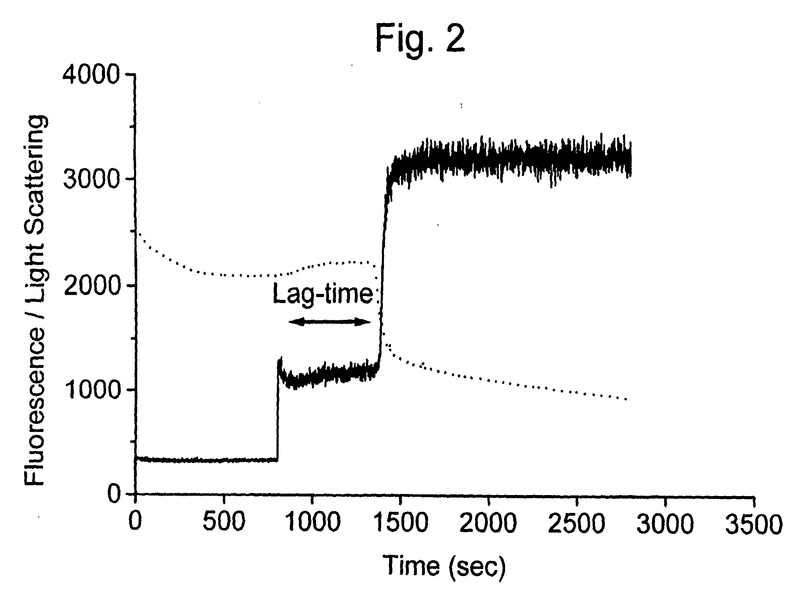

Fig. 2. Characteristic reaction time profile at 41 °C for phospholipase A₂, PLA₂, (A. piscivorus piscivorus) hydrolysis of unilamellar liposomes composed of 1-O-DPPC. The PLA₂ hydrolysis reaction is monitored by intrinsic fluorescence (solid line) from the enzyme and 90° static light scattering (dashed lines) from the lipid suspension. After adding PLA₂, at 800 sec to the equilibrated liposome suspension a characteristic lag-time follows before a sudden increase in the catalytic activity takes place. Samples for HPLC were taken before adding the enzyme and 20 minutes after the observed lag time.

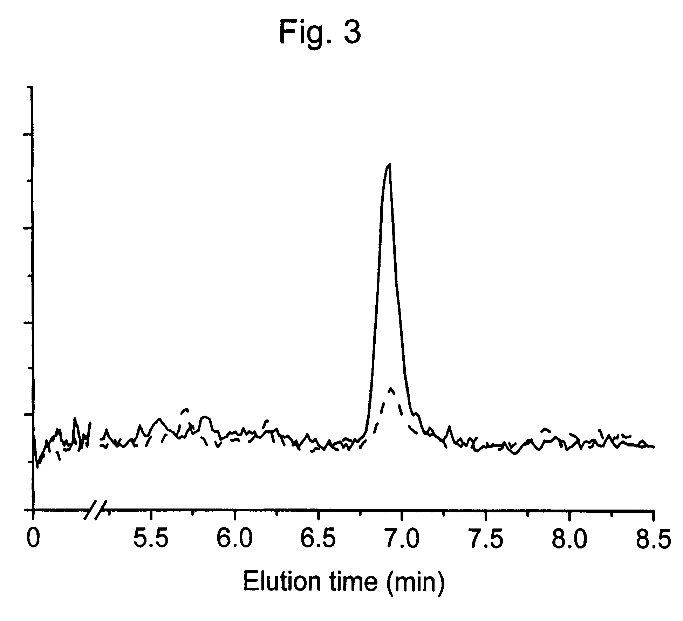

Fig. 3. HPLC chromatograms illustrating the effect of phospholipase A₂ hydrolysis of liposomes composed of 1-O-DPPC. The chromatograms show the amount of 1-O-DPPC (100%, solid line) before phospholipase A₂ (A. piscivorus piscivorus) was added to the liposome suspension and the amount of 1-O-DPPC (21%, dashed line) after the lag-burst.

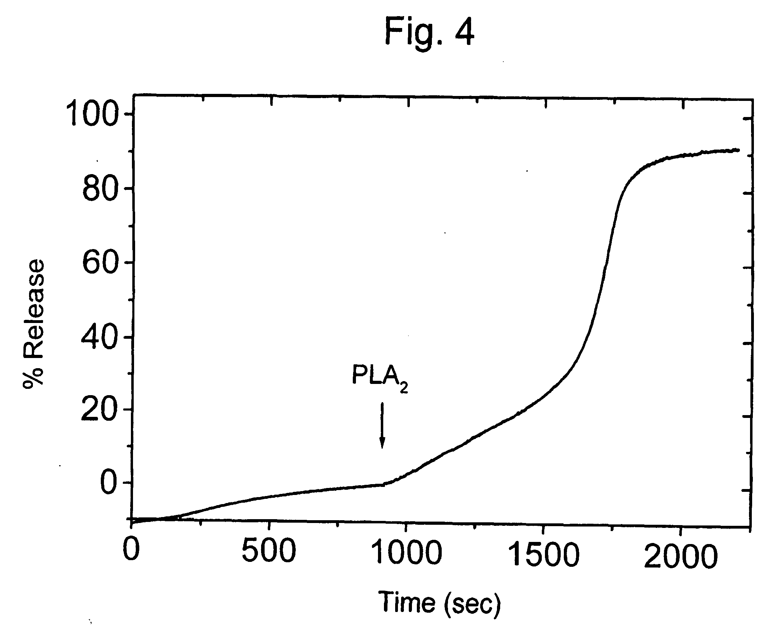

Fig. 4. PLA₂-controlled release of the fluorescent model drug calcein from liposomes composed of 25 µM 1-O-hexadecyl-2-hexadecanoyl-*sn*-glycero-3-phosphocholine (1-O-DPPC) suspended in a 10 mM HEPES-buffer (pH = 7.5), as a function of time. 25 nM phospholipase A₂ (A. piscivorus piscivorus) was added at time 900 sec, the temperature was 37°C. The percentage of calcein released is determined as % Release =100 (I_{F(t)}-I_{B})/(I_{T} - I_{B}), where I_{F(t)} is the measured fluorescence at time t after addition of the enzyme, I_{B} is the background fluorescence, and I_{T} is the total fluorescence measured after addition of Triton X-100 which leads to complete release of calcein by breaking up the liposomes.

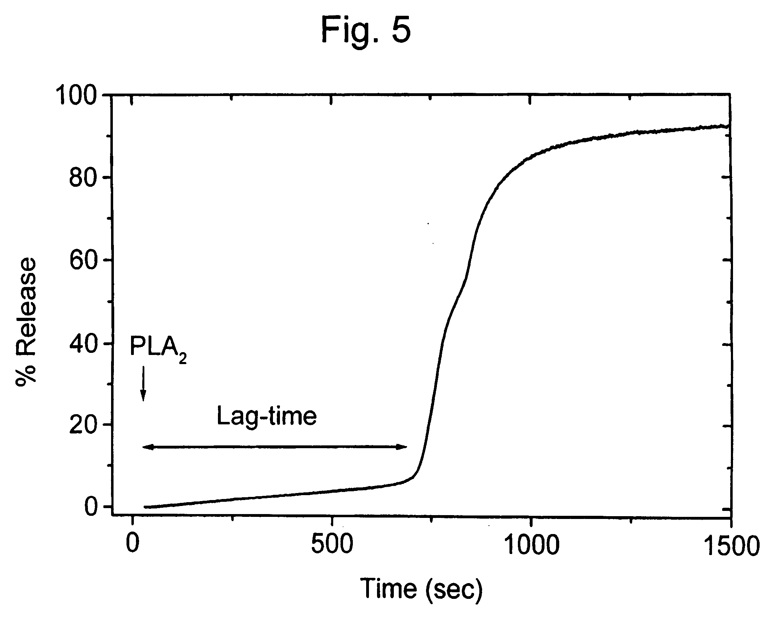

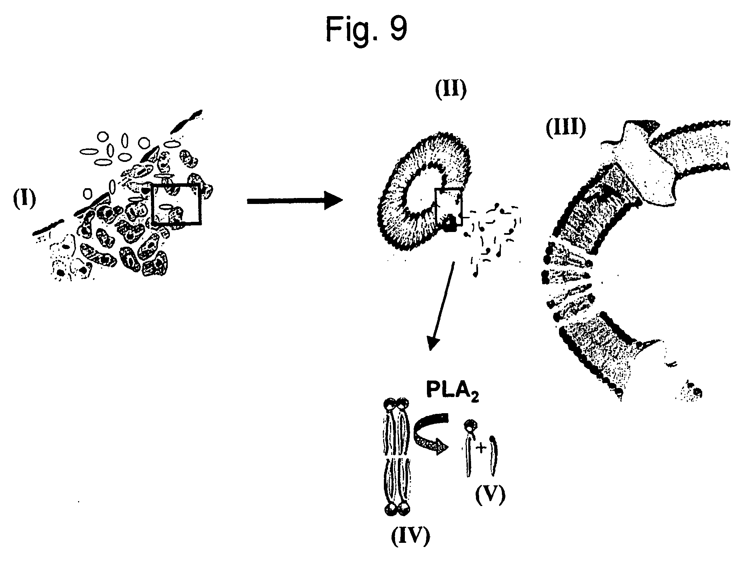

Fig. 5. PLA₂-controlled release of the fluorescent model drug calcein across the target membrane of non-hydrolysable membranes (see Fig. 9), as a function of time for liposomes composed of 25 µM 1-O-hexadecyl-2-hexadecanoyl-*sn*-glycero-3-phosphocholine (1-O-DPPC) suspended in a 10 mM HEPES-buffer (pH = 7.5). 25 nM phospholipase A₂ was added at time 0 sec and the temperature was 37°C. The percentage of calcein released is determined as described in Fig. 4.

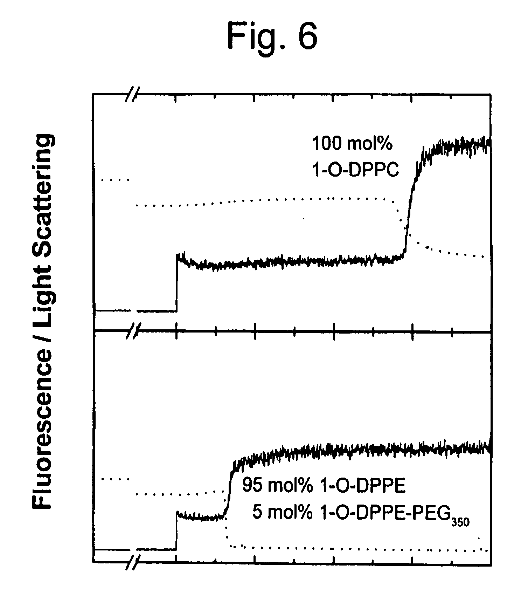

Fig. 6. Characteristic reaction time profiles at 41°C for PLA₂ (A. piscivorus piscivorus) hydrolysis of unilamellar liposomes incorporated with 0 and 5% negatively charged 1-O-DPPE-PEG350 lipopolymers. The PLA₂ hydrolysis reaction is monitored by intrinsic fluorescence (solid line) from the enzyme and 90° static light scattering (dashed lines) from the suspension. After adding PLA₂ to the equilibrated liposome suspension a characteristic lag-time follows before a sudden increase in the catalytic activity takes place.

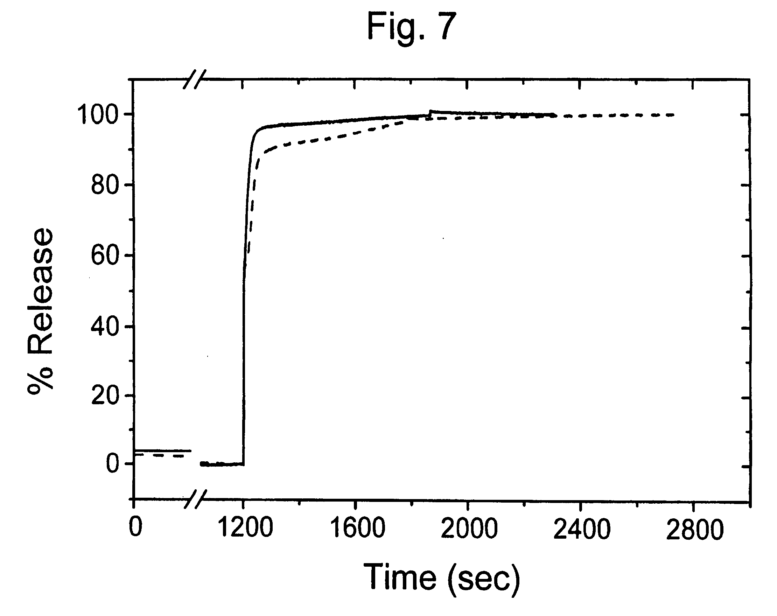

Fig. 7. PLA₂-controlled release of the fluorescent model drug calcein across the target membrane of non-hydrolysable D-O-SPC membranes as a function of time for micelles composed of 25 µM 1-O-DPPE-PEG350 (dotted line), DSPE-PEG750 (dashed line), 1-O-DPPE-PEG2000 (solid line) suspended in a 10 mM HEPES-buffer (pH = 7.5). Phospholipase A₂ (25 nM) was added at time 1200 sec and the temperature was 41°C. The percentage of calcein released is determined as described in Fig. 4. PLA₂ catalysed hydrolysis of 1-O-DPPE-PEG350 induced the fastest and highest release.

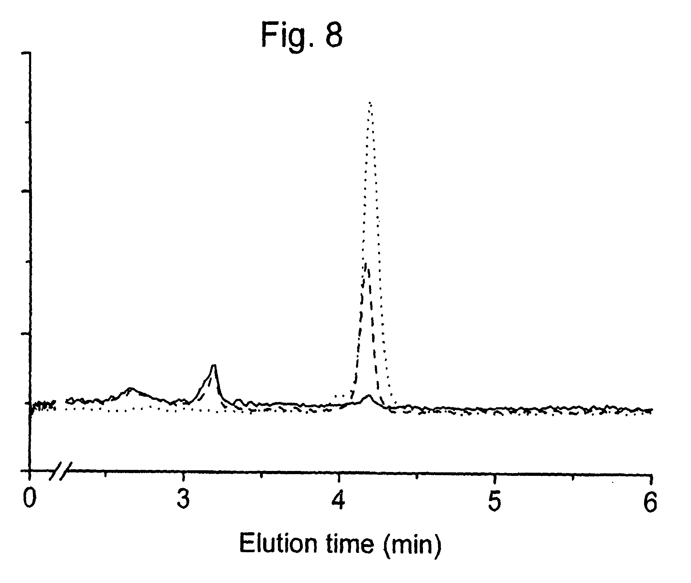

Fig. 8. HPLC chromatograms illustrating the effect of phospholipase A₂ hydrolysis of micelles composed DSPE-PEG750 (0.150 mM). The chromatograms show the amount of stearic acid generated before (solid line) phospholipase A₂ (A. piscivorus piscivorus) was added to the micelle suspension and the amount (dashed line) of DSPE-PEG750 after the lag-burst. The dotted line show pure stearic acid (0.4 mM). The percentage hydrolysis was calculated on basis of the integrated area of the stearic acid standard (115850 units) and the integrated area of the sample (45630 units). The concentration of the stearic acid in the sample was calculated to (45630/115850 x 0.4 mM) 0.157 mM, which means that 100% of the DSPE-PEG750 was hydrolysed to lyso-DSPE-PEG750 and stearic acid.
Fig. 9. Describes the principle of flexible prodrug liposome targeting, release and absorption by extracellular enzymes.
   (I) Stratum corneum with small pores
   (II) Flexible prodrug liposome
   (III) Target cell and cell membrane
   (IV) Prodrug (i.e. monoether-lipid), proenhancer (lipid), proactivator (lipid)
   (V) Drugs (i.e. ether-lysolipid and fatty acid derivatives) enhancers (lysolipid + fatty acid) PLA₂ activators (lysolipid + fatty acid)

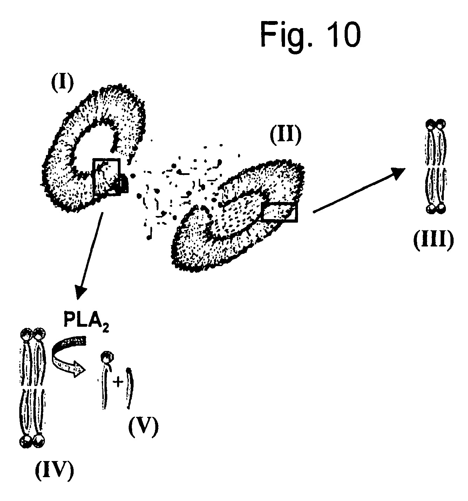

Fig. 10. Schematic illustration of a liposomal drug-targeting principle involving transport of the flexible liposomal drug carriers into the diseased tissue and subsequent release of drug and transport across the target membrane via extracellular PLA₂ activity.
   (I) Flexible prodrug carrier liposome
   (II) Non-degradable target liposomal membrane
   (III) Non-hydrolysable ether-lipids
   (IV) Proenhancer (lipid), prodrug (i.e. monoether-lipid), proactivator (lipid)
   (V) Enhancers (lysolipid + fatty acid), drugs (i.e. ether-lysolipid and fatty acid derivatives), PLA₂ activators (lysolipid + fatty acid)

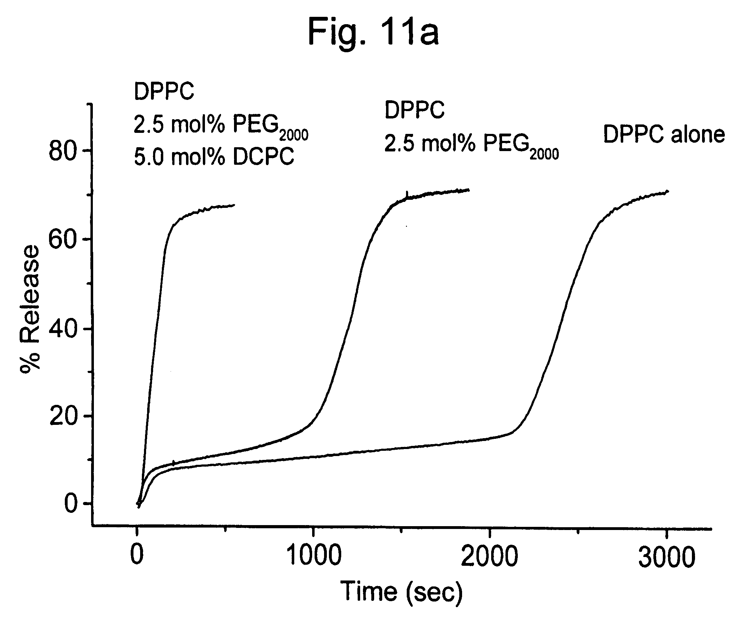

Fig. 11 (a) PLA₂-controlled release of the fluorescent model drug calcein across the target membrane as a function of time for different compositions of the carrier liposomes. The temperature is 37°C. In comparison with bare DPPC carriers, the rate of release of the model drug is dramatically enhanced for the negativly charged carriers, DPPC + 2.5 mol% 1-O-hexadecyl-2-hexadecanoyl-*sn*-glycero-3-phosphoethanolamine-N-[methoxy(polyethylene glycol)-2000] (DPPE-PEG2000). A further augmentation of the rate of release is obtained if the carrier also contains a short-chain phospholipid, didecanoylphosphatidylcholine (DCPC), which acts as a local activator for the enzyme. The percentage of calcein released is determined as % Release =100 (I_{F(t)}-I_{B})/(I_{T} - I_{B}), where I_{F(t)} is the measured fluorescence at time t after addition of the enzyme, I_{B} is the background fluorescence, and I_{T} is the total fluorescence measured after addition of Triton X-100 which leads to complete release of calcein by breaking up the target liposomes. (b) PLA₂-controlled release of the fluorescent model drug calcein across the target membrane as a function of time for different temperatures. As the temperature is raised, the rate of release is enhanced due to increased activity of the enzyme induced by structural changes in the lipid bilayer substrate of the carrier liposome. In the present assay a maximum release of about 70% is achieved in all cases. The insert shows the time of 50% calcein release, t_{50%}, as a function of temperature. The concentration of the target and carrier liposomes are 25 µM, and PLA₂ is added in a 25 nM concentration in a HEPES buffer with pH=7.5.

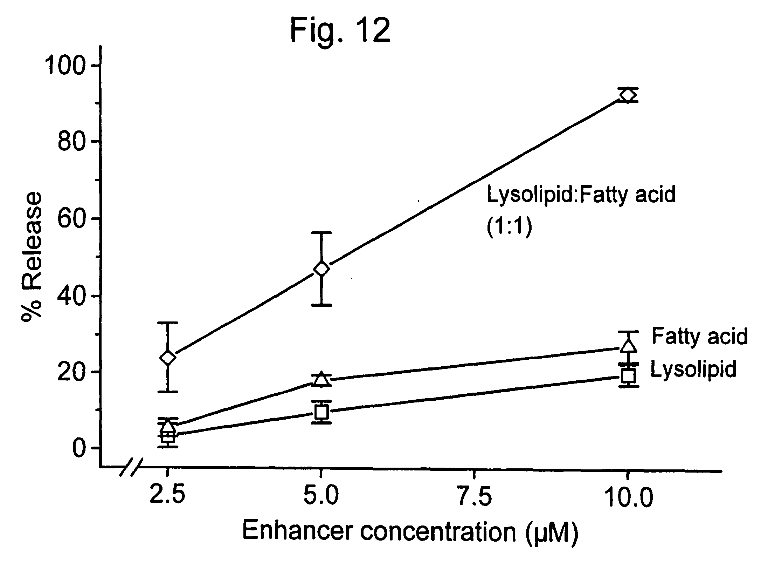

Fig. 12. Total release after 20 min of the fluorescent model drug calcein across the target membrane as a function of adding increasing amounts of lyso-palmitoyl phospholipid and palmitic acid, separately, and in a 1:1 mixture. The concentration of the target membranes is 25 µM in a HEPES buffer with pH=7.5 at a temperature of 39°C.

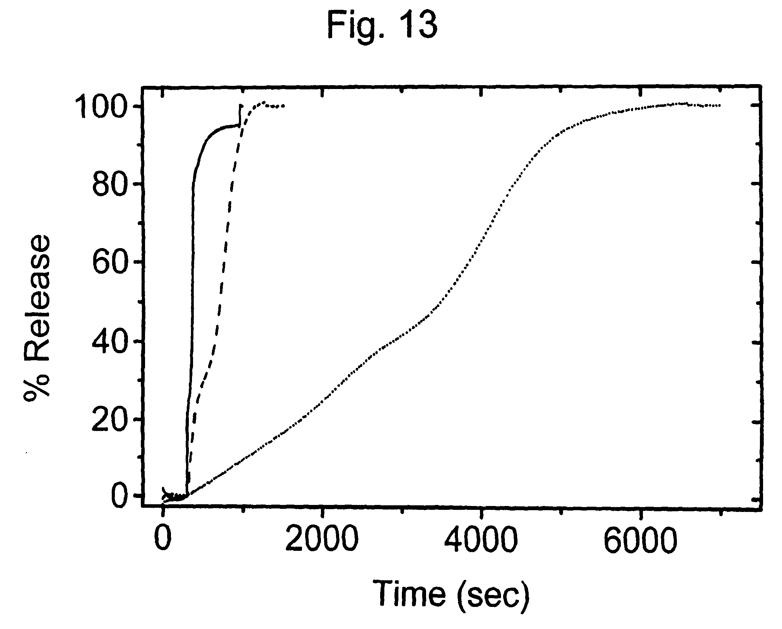

Fig 13. PLA₂-controlled release of the fluorescent model drug calcein from liposomes composed of 25 µM 90 mol% 1-O-DPPC and 10 mol% of the negatively charged 1-O-DPPE-PEG350 lipid suspended in a 10 mM HEPES-buffer (pH = 7.5), as a function of time. 50 nM (straight line), 1 nM (solid line) and 0.02 nM (dotted line) phospholipase A₂ (A. piscivorus piscivorus) was added at time 300 sec, the temperature was 35.5°C. The percentage of calcein released is determined as describe in Fig 4.

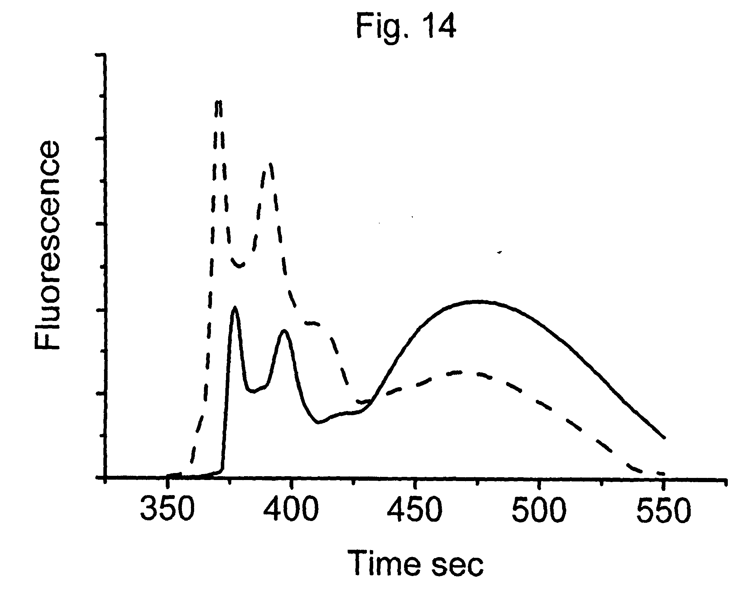

Fig. 14. Emission spectrum of 1 mol% bis-py-DPC incorporated into negatively charged liposomes (0.100 mM) before (solid line) and after (dashed line) adding 100 nM PLA₂ (*Agkistrodon piscivorus piscivorus)* to the liposome suspension equilibrated at 41 °C.

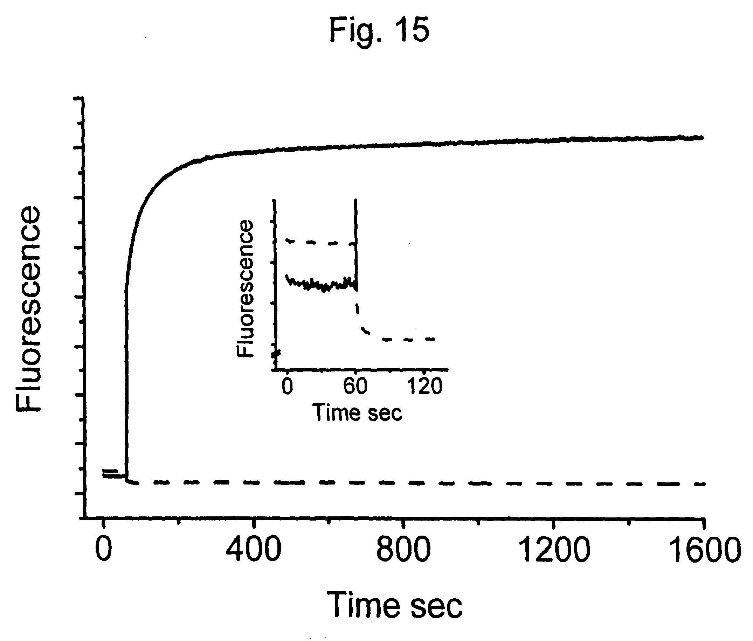

Fig. 15. Characteristic reaction time profile at 41°C for rat phospholipase catalysed hydrolysis of negatively charged liposomes. The catalytic reaction was initiated by adding cell-free peritoneal fluid to 2.5 ml of the thermostated liposome suspension equilibrated for 60 sec prior to addition of the peritoneal fluid. The hydrolysis reaction is monitored by monomer fluorescence (solid line) and eximer fluorescence (dashed line) from bis-py-DPC. After adding undiluted peritoneal fluid, at 60 sec, to the equilibrated liposome suspension a sudden increase in monomer fluorescence, and a simultaneously decrease in eximer fluorescence is observed as the bis-py-DPC substrate is hydrolysed. The insert shows the reaction time profile of the first 120 sec.

### DETAILED DESCRIPTION OF THE INVENTION

The major function of the skin is to protect the individual from the environment. The mammalian skin is a complex organ consisting of three well-defined layers, the outermost epidermis layer followed by the dermis and the subcutis. When used herein the term "skin" is intended to be synonymous with the term "cutaneous and subcutaneous tissue". The major physico-chemical barrier of skin is localized in the outermost cornified layer of the skin, the stratum comeum. The stratum corneum is the most specialized structure of the skin. It is the end product of the differentiation process of the epidermis. The majority of the cells of the epidermis consist of keratinocytes in various states of differentiation. The lowermost keratinocytes, the basal cells, reside on a basal membrane in contact with the dermis, that is the connective tissue of the skin, and are the only keratinocytes that have dividing capability. A fraction of the basal cells continuously leaves the basal membrane and goes through a differentiation process which eventually makes the cells become building blocks of the stratum comeum. In this process the keratinocytes go through a number of adaptive changes. There is an increased content of cytoskeleton consisting of epidermis-specific cytokeratins. The intermediate filaments of contiguous cells are joined to a functional unit by an increased number of desmosomes. The most dramatic changes take place during the transition from the uppermost living cell layer, the stratum granulosum, to the non-viable stratum corneum in a process usually called keratinization. The result of the differentiation is the non-viable cells of the stratum corneum, the corneocytes. The individual comeocytes cells are held together by mechanically resistant structures, the desmosomes, and forms the microporous physico-chemical barrier of the skin. The pores in the stratum corneum and underlaying structures are normally so narrow, that the skin only allow passage of entities smaller than 400 Da, but lipid vesicles that are able to pass pores smaller than 30 nm are able to penetrate skin (Cevc et al. (Biochem Biophys Acta (1998) 1368, p 201-215, US Patent 6.180.353).

However, in order to provide the active substance to the relevant target cells permeability is only one part of the challenge. As important as getting the substance into the body is the issue of ensuring that predominantly the relevant cells are exposed to the pharmaceutical active component. The present invention discloses a novel drug delivery systems that ensures that the pharmaceutical active component(s) can be delivered specifically at the target cells in the skin.

In particular the present invention addresses both the important issue of ensuring that predominantly the relevant cells are exposed to the pharmaceutical active component, and the fact that only very flexible liposomes that contains "edge active substances" (see below) are able to pass through the stratum cormeum and penetrate deep into the skin or mucous membranes of humans or animals (hereafter "skin"). It is important to realise that the present invention is not intended for application of compound into the blood-stream via the skin.

It has been found that the activity of extracellular phospholipase A₂ (PLA₂) is increased in a number of inflammatory diseases of the skin most notably psoreasis and eczema (Forster et al. (1985) Br J Dermatol 112:135-47; Forster et al. (1983) Br J Dermatol 108:103-5). It has furthermore been found that extracellular PLA₂ is capable of cleaving monoether/monoester lipid derivatives so as to produce monoether lysolipid derivatives which as such, or in combination with other active compounds, will exhibit a therapeutic effect. Thus the present invention is based on the notion that the pharmaceutical active component(s) can be delivered specifically at the target cells in the skin taking advantage of an elevated activity of PLA₂ in the part of the tissue harboring the target cells. In one particular embodiment of the invention a specific lipid-analogue compound may be incorporated into the carrier liposome and act as a prodrug which is turned into an active drug by hydrolysis via the PLA₂. A possible example could be therapeutically active compounds (e.g. regulatory fatty acid derivatives and other lipophilic groups, e.g. vitamin derivatives, steroid derivatives, etc. such as cholecalciferol and tocopherol analogues) that is ester bound to the phospholipid in the sn-2 position and therefore renders the lipid derivative a substrate for PLA₂.

The possibility of including the pharmaceutically active substance in the liposomes as "second drug substances" - see below - seem particular interesting in relation with dermal application.

Thus the invention disclosed herein offers a solution to both the issue of penetration of intact skin and mucous membranes and the issue of specific targeting of cells, tissues or part of tissues that are characterized by an increased level of PLA₂.

### Lipid derivatives

The drug delivery systems (liposomes) for use in the present invention relies on lipid derivative having (a) an aliphatic group of a length of at least 7 carbon atoms and an organic radical having at least 7 carbon atoms, and (b) a hydrophilic moiety, said prodrug furthermore being a substrate for extracellular PLA₂ to the extent that the organic radical can be hydrolytically cleaved off, whereas the aliphatic group remains substantially unaffected, whereby the active drug substance is liberated in the form of a lysolipid derivative which is not a substrate for lysophospholipase.

Although the terms "lipid" and "lysolipid" (in the context of phospholipids) will be well-known terms for the person skilled in the art, it should be emphasised that, within the present description and claims, the term "lipid" is intended to mean triesters of glycerol of the following formula: 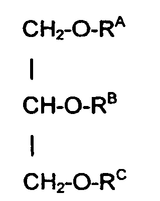 wherein R^{A} and R^{B} are fatty acid moieties (C₉₋₃₀-alkyl/alkylene/alkyldiene/alkyltriene/alkyltetraene-C(=O)-) and R^{C} is a phosphatidic acid (PO₂-OH) or a derivative of phosphatidic acid. Thus, the groups R^{A} and R^{B} are linked to the glycerol backbone via ester bonds.

The term "lysolipid" is intended to mean a lipid where the R^{B} fatty acid group is absent (e.g. hydrolytically cleaved off), i.e. a glycerol derivative of the formula above where R^{B} is hydrogen, but where the other substituents are substantially unaffected. Conversion of a lipid to a lysolipid can take place under the action of an enzyme, specifically under the action of cellular as well as extracellular PLA₂.

The terms "lipid derivative" and "lysolipid derivative" are intended to cover possible derivatives of the above possible compounds within the groups "lipid" and "lysolipid", respectively. Examples of biologically active lipid derivatives and lysolipid derivatives are given in Houlihan, et al., Med. Res. Rev., 15, 3, 157-223. Thus, as will be evident, the extension "derivative" should be understood in the broadest sense.

Within the present application, lipid derivatives and lysolipids should however fulfil certain functional criteria (see above) and/or structural requirements. It is particularly relevant to note that the suitable lipid derivatives are those which have (a) an aliphatic group of a length of at least 7, preferably at least 9, carbon atoms and an organic radical having at least 7 carbon atoms, and (b) a hydrophilic moiety. It will be evident that the aliphatic group and the organic radical will correspond to the two fatty acid moieties in a normal lipid and that the hydrophilic moiety will correspond to the phosphate part of a (phospho)lipid or a bioisoster thereof.

Thus, as the general idea behind the present invention is to exploit the increased level of extracellular PLA₂ activity in localised areas cutaneous and subcutaneous tissue, the lipid derivatives which can be utilised within the present invention should be substrates for extracellular PLA₂, i.e. the lipid derivatives should be able to undergo hydrolytic, enzymatic cleavage of the organic radical corresponding to the fatty acid in the 2-position in a lipid. Extracellular PLA₂ is known to belong to the enzyme class (EC) 3.1.1.4. Thus by reference to (extracellular) PLA₂ should be understood all extracellular enzymes of this class, e.g. lipases, which can induce hydrolytic cleavage of the organic radical corresponding to the fatty acid in the 2-position in a lipid. One particular advantage of the lipid based drug delivery system (as liposomes) is that extracellular PLA₂ activity is significantly increased towards organised substrates as compared to monomeric substrates.

In view of the requirement to hydrolysability by extracellular PLA₂, it is clear that the organic radical (e.g. aliphatic group) is preferably linked via an ester functionality which can be cleaved by extracellular PLA₂, preferably so that the group which is cleaved off is a carboxylic acid.

Furthermore, an important feature is that the aliphatic group (the group corresponding to the fatty acid in the 1-position in a lipid) of the lipid derivative, i.e. the lysolipid derivative after cleavage by extracellular PLA₂, is substantially unaffected by the action of extracellular PLA₂. By "substantially unaffected" is meant that the integrity of the aliphatic group is preserved and that less than 1 mol%, preferably less than 0.1 mol%, of the aliphatic group (the aliphatic group in the 1-position) is cleaved under the action of lysophospholipase.

Also, the lysolipid derivative resulting from the hydrolytic cleavage of the organic radical should not in itself be a substrate for lysophospholipase. Lysophospholipase is known to belong to the enzyme class (EC) 3.1.1.5. Thus by reference to lysophospholipase should be understood all enzymes of this class that catalyses the reaction lyso(phospho)lipid + water yielding phosphoglycerol + fatty acid. The term "not a substrate for lysophospholipase" is intented to mean that lysophospholipase has an activity of less than 1% towards the substrate compared with the corresponding esterlipid, i.e. virtually not enzymatic activity.

Suitable examples of such lysolipid derivatives are those which will not undergo hydrolytical cleavage under the action of lysophospholipases. Thus, the lysolipid derivatives are in particular not lysolipids and lysolipid derivatives which have an ester linkage in the 1-position of the lysolipid or the position of a lysolipid derivative corresponding to the 1-position of a lysolipid.

One preferred class of lipid derivatives for incorporation in the drug delivery systems can be represented by the following formula: 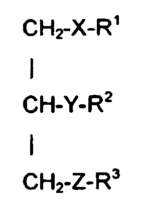 wherein
X and Z independently are selected from O, CH₂, NH, NMe, S, S(O), and S(O)₂, preferably from O, NH, NMe and CH₂, in particular O;
Y is -OC(O)-, Y then being connected to R² via either the oxygen or carbonyl carbon atom, preferably via the carbonyl carbon atom;
R¹ is an aliphatic group of the formula Y¹Y²;
R² is an organic radical having at least 7 carbon atoms, such as an aliphatic group having a length of at least 7, preferably at least 9, carbon atoms, preferably a group of the formula Y¹Y²;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl, but preferably Y¹-Y² is unsubstituted,
R³ is selected from phosphatidic acid (PO₂-OH), derivatives of phosphatidic acid and bioisosters to phosphatic acid and derivatives thereof.

As mentioned above, preferred embodiments imply that Y is -OC(O)- where Y is connected to R² via the carboxyl atom. The most preferred embodiments imply that X and Z are O and that Y is -OC(O)- where Y is connected to R² via the carboxyl atom. This means that the lipid derivative is a 1-monoether-2-monoester-phospholipid type compound.

Another preferred group of lipid derivatives is the one where the group X is S.

In one embodiment, R¹ and R² are aliphatic groups of the formula Y¹-Y² where Y² is CH₃ or CO₂H, but preferably CH₃, and where Y¹ is -(CH₂)ₙ₁(CH=CH)ₙ₂(CH₂)ₙ₃(CH=CH)ₙ₄-(CH₂)ₙ₅(CH=CH)ₙ₆(CH₂)ₙ₇(CH=CH)ₙ₈(CH₂)ₙ₉; the sum of n1+2n2+n3+2n4+n5+2n6+n7+ 2n8+n9 is an integer of from 9 to 23; that is, the aliphatic group, Y¹Y², is from 10-24 carbon atoms in length. n1 is equal to zero or is an integer of from 1 to 23; n3 is equal to zero or is an integer of from 1 to 20; n5 is equal to zero or is an integer of from 1 to 17; n7 is equal to zero or is an integer of from 1 to 14; n9 is equal to zero or is an integer of from 1 to 11; and each of n2, n4, n6 and 8 is independently equal to zero or 1.

Although the aliphatic groups may be unsaturated and even substituted with halogens (flouro, chloro, bromo, iodo) and C₁₋₄-groups (i.e. yielding branched aliphatic groups), the aliphatic groups as R¹ and R² are in one embodiment preferably saturated as well as unbranched, that is, they preferably have no double bonds between adjacent carbon atoms, each of n2, n4, n6 and n8 then being equal to zero. Accordingly, Y¹ is preferably (CH₂)ₙ₁. More preferably (in this embodiment), R¹ and R² are each independently (CH₂)ₙ₁CH₃, and most preferably, (CH₂)₁₇CH₃ or (CH₂)₁₅CH₃. In alternative embodiments, the groups can have one or more double bonds, that is, they can be unsaturated, and one or more of n2, n4, n6 and n8 can be equal to 1. For example, when the unsaturated hydrocarbon has one double bond, n2 is equal to 1, n4, n6 and n8 are each equal to zero and Y¹ is (CH₂)ₙ₁ CH=CH(CH₂)ₙ₃. n1 is equal to zero or is an integer of from 1 to 21, and n3 is also zero or is an integer of from 1 to 20, at least one of n1 or n3 not being equal to zero.

In one particular embodiment, the lipid derivatives are those which are monoether lipids where X and Z are O, R¹ and R² are independently selected from alkyl groups, (CH₂)ₙCH₃, where n is 11, 12, 13, 14, 15, 16, 17, 18, 19, 20, 21, 22, 23, 24, 25, 26, 27, 28, or 29, preferably 14, 15 or 16, in particular 14; Y is -OC(O)-, Y then being connected to R² via the carbonyl carbon atom.

With respect to the hydrophilic moiety (often known as the "head group") which corresponds to R³, it is believed that a wide variety of groups corresponding to phosphatidic acid (PO₂-OH), derivatives of phosphatidic acid and bioisosters to phosphatic acid and derivatives thereof can be used. As will be evident, the crucial requirement to R³ is that the groups should allow for enzymatic cleavage of the R² group (actually R²-C(=O) or R²-OH) by extracellular PLA₂. "Bioisosters to phosphatidic acid and derivatives thereof" indeed implies that such groups - as phosphatidic acid - should allow for enzymatic cleavage by extracellular PLA₂.

R³ is typically selected from phosphatidic acid (PO₂-OH), phosphatidylcholine (PO₂-O-CH₂CH₂N(CH₃)₃), phosphatidylethanolamine (PO₂-O-CH₂CH₂NH₂), N-methylphosphatidylethanolamine (PO₂-O-CH₂CH₂NCH₂), phosphatidylserine, phosphatidylinositol, and phosphatidylglycerol (PO₂-O-CH₂CHOHCH₂OH). Other possible derivatives of phosphatidic acid are those where dicarboxylic acids, such as glutaric, sebacic, succinic and tartaric acids, are coupled to the terminal nitrogen of phosphatidylethanolamines, phosphatidylserine, phosphatidylinositol, etc.

One highly interesting aspect is the possibility of modifying the pharmaceutical effect of the lipid derivative by modifying the group R². It should be understood that R² should be an organic radical having at least 7 carbon atoms) (such as an aliphatic group having a certain length (at least 7, preferably 9, carbon atoms)), a high degree of variability is possible, e.g. R² need not necessarily to be a long chain residue, but may represent more complex structures.

Generally, it is believed that R² may either be rather inert for the environment in which it can be liberated by extracellular PLA₂ or that R² may play an active pharmaceutical role, typically as an auxiliary drug substance for the treatment of skin conditions or as an efficiency modifier for the lysolipid derivative and/or any other (second) drug substances present in the environment.

In some embodiments, the group R² will be a long chain residue, e.g. a fatty acid residue (the fatty acid will include a carbonyl from the group Y). This has been described in detail above. Interesting examples of auxiliary drug substances as R² within this subgroups are polyunsaturated acids, e.g. oleate, linoleic, linonleic, as well as derivatives of arachidonoyl (including the carbonyl from Y), e.g. prostaglandins such as prostaglandin E₁, as arachidonic acid derivatives are know regulators of hormone action including the action of prostaglandins, thromboxanes, and leukotrines. Examples of efficiency modifiers as R² are those which enhance the permeability of the target cell membrane as well as enhances the activity of extracellular PLA₂ or the active drug substance or any second drug substances. Examples hereof are short chain (C₈₋₁₂) fatty acids.

However, it is also envisaged that other groups might be useful as the organic radical R², e.g. vitamin D derivatives, steroid derivatives, retinoic acid (including all-trans-retinoic acid, all-cis-retinoic acid, 9-cis-retinoic acid, 13-cis-retinoic acid), cholecalciferol and tocopherol analogues, pharmacologically active carboxylic acids such as branched-chain aliphatic carboxylic acids (e.g. valproic acid and those described in WO 99/02485), salicylic acids (e.g. acetylsalicylic acid), steroidal carboxylic acids (e.g. lysergic and isolysergic acids), monoheterocyclic carboxylic acids (e.g. nicotinic acid) and polyheterocyclic carboxylic acids (e.g. penicillins and cephalosporins), diclofenac, indomethacin, ibuprofen, naproxen, 6-methoxy-2-naphthylacetic acid as well as fatty acid derivatives such as N-(ω-3)-fatty acid derivatives.

It should be understood that the various examples of possible R² groups are referred to by the name of a discrete species, rather than the name of the radical. Furthermore, it should be understood that the possible examples may include the carbonyl group or oxy group of the bond via which the organic radical is linked to the lipid skeleton (corresponding to "Y" in the formula above). This will of course be appreciated by the person skilled in the art.

Even though it has not specifically been indicated in the general formula for the suitable examples of lipid derivatives to be used within the present invention, it should be understood that the glycol moiety of the lipid derivatives may be substituted, e.g. in order to modify the cleavage rate by extracellular PLA₂ or simply in order to modify the properties of the liposomes comprising the lipid derivatives.

### Lipid derivatives as prodrugs

As described above, the present invention provides the use of a lipid-based drug delivery system for the preparation of a medicament for treatment or prevention of treatment of diseases or conditions associated with a localised increase in extracellular PLA₂ activity in skin of a mammal in the form of a liposome for administration of an active drug substance selected from lysolipid derivatives, said system comprising an edge active compound, and said active drug substance is present in the lipid-based system in the form of a prodrug, said prodrug being a lipid derivative having (a) an aliphatic group of a length of at least 7 carbon atoms and an organic radical having at least 7 carbon atoms, and (b) a hydrophilic moiety, said prodrug furthermore being a substrate for extracellular PLA₂ to the extent that the organic radical can be hydrolytically cleaved off, whereas the aliphatic group remains substantially unaffected, whereby the active drug substance is liberated in the form of a lysolipid derivative which is not a substrate for lysophospholipase.

By the term "active drug substance" is meant any chemical entity which will provide a prophylactic or therapeutic effect in the body of a mammal, in particular a human, with special respect to skin diseases. Also substances that may find use for cosmetic purposes are here considered a "active drug substance".

The term "prodrug" should be understood in the normal sense, namely as a drug which is masked or protected with the purpose of being converted (typically by cleavage, but also by in vivo chemical conversion) to the intended drug substance. The person skilled in the art will recognise the scope of the term "prodrug". Also substances that may find use for cosmetic purposes are here concidered a "prodrug".

The active drug substance is selected from lysolipid derivatives, and as it will be understood from the present description with claims, the lysolipid derivatives relevant within the present invention will have a therapeutic effect - at least - in connection with the diseases and conditions specified herein, i.e. particular diseases and conditions where a local area of the body of the mammal has a level of extracellular PLA₂ activity which can liberate the lysolipid derivative.

As will be understood from the present description with claims, the lipid derivative will often constitute the prodrug referred to above and the lysolipid derivative will thereby constitute the active drug substance often a monoether lysolipid derivative. It should however be understood that this does not exclude the possibility of including other drug substances, referred to as second drug substances, in the drug delivery systems, neither does it exclude that the organic radical which can be hydrolytically cleaved by the action of extracellular PLA₂ can have a certain pharmaceutical effect (e.g. as an auxiliary drug substance or an efficiency modifier as described elsewhere herein). Furthermore, the pharmaceutical effect of the "active drug substance", i.e. the lysolipid derivative, need not be the most predominant when a second drug substance is included, actually the effect of the second drug substance might very well be the most predominant as will become apparent in the other main embodiment (see "Lipid derivative liposomes as drug delivery systems", below).

The active drug substance (lysolipid derivative) released from the prodrug (lipid derivative) is believed to take place as illustrated in the following example:

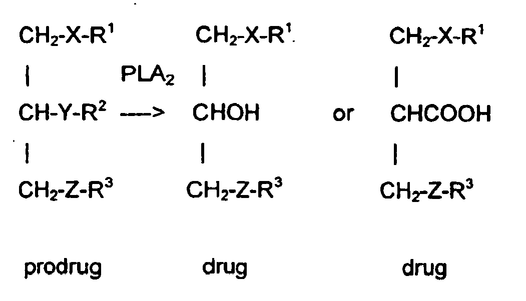

Furthermore, the substituent R² may constitute an auxiliary drug substance or an efficiency modifier for the active drug substance and will simultaneously be released under the action of extracellular PLA₂:

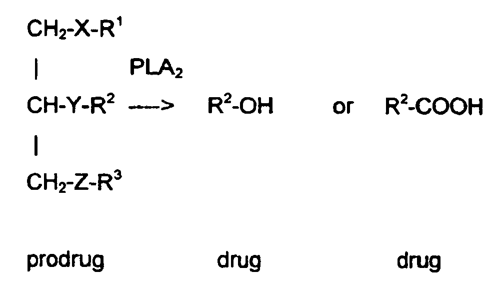

It has been described above under the definition of R² how the group R² can have various independent or synergistic effects in association with the active drug substance, e.g. as an auxiliary drug substance or an efficiency modifier, e.g. permeability or cell lysis modifier. It should be borne in mind that the groups corresponding to R² (e.g. R²-OH or R²-COOH) might have a pharmaceutical effect which is predominant in relation the effect of the lysolipid derivative (active drug substance).

As is evident to those skilled in the art the criteria for effect is less stringent for components of cosmetic formulations.

### Lipid derivatives formulated as liposomes

The term "lipid-based drug delivery system in the form of a liposome" should encompass macromolecular structures which as the main constituent include lipid or lipid derivatives and which at the same time are able to undergo sufficient deformation to enable the structure to pass through the stratum corneum and deep into the skin of a mammal. A preferred example hereof is flexible liposomes (e.g. Transfersomes^{®}) as described by the company IDEA AG, Germany, in US 6,165,500.

In one important variant which advantageously can be combined with the embodiments described herein, the lipid derivative (e.g. the prodrug) is included in liposomes in combination with other constituents (surfactants, other lipids, agents, etc.) in such a way that the liposome becomes sufficiently flexible. Thus, the lipid-based systems described herein are preferably in the form of liposomes, wherein the liposomes are build up of layers comprising the lipid derivative (e.g. a prodrug).

Other important constituents of liposomes, namely the constituents providing the flexible properties to the liposomes, can generally be characterised as pharmaceutically acceptable "edge active substances", namely such constituents that will "smoothen" or "soften" the lipid membrane.

An "edge active substance" according to this application is any substance which is capable of inducing or increasing the carrier system's capacity to form edges, protrusions or relatively strongly curved surfaces; this property also manifests itself in the capability to induce pores in lipid structures, such as membranes, or even provoke a solubilization (lysis) in the higher concentrations ranges. More strictly speaking, all such substances are considered edge-active which exhibit a tendency to accumulate at or near the edges between the polar and apolar parts of molecules and/or near or at the edges between the polar and apolar parts of the supramolecular aggregates, thereby lowering the free energy for the formation of edges and/or strongly curved surfaces. All surfactants and many solvents as well as asymmetric, and thus amphiphatic, molecules or polymers, such as many oligo- and polycarbohydrates, oligo- and polypeptides, oligo- and polynucleotides or their derivatives also belong to this category. The surfactant or surfactant-like material preferrably is a nonionic, a zwitterionic, an anionic or a cationic surfactant, especially a fatty-acid oralcohol, an alkyl-tri/di/methyl-ammonium salt, an alkylsulphate salt, a monovalent salt of cholate, deoxycholate, glycocholate, glycodeoxycholate, taurodeoxycholate, taurocholate, etc., an acyl-or alkanoyl-dimethylaminoxide, esp. a dodecyl-dimethyl-aminoxide, an alkyl-or alkanoyl-Nmethylglucamide, N-alkyl-N, N-dimethylglycine, 3(acyldimethylammonio)-alkanesulphonate, N-acyl-sulphobetaine, a polyethylene-glycol-octylphenyl ether, esp. a nonaethylene-glycoloctylphenyl ether, a polyethylene-acyl ether, esp. a nonaethylen-dotiecyl ether, a polyethylene-glycol-isoacyl ether, esp. a octaethylene-glycolisotridecyl ether, polyethylene-acyl ether, esp. octaethylenedodecyl ether, polyethylene-glycol-sorbitane-acyl ester, such as polyethylenglykol-20monolaurate (Tween 20) or polyethylenglykol-20-sorbitan-monooleate (Tween 80), a polyhydroxyethylene-acyl ether, esp. polyhydroxyethylenelauryl,-myristoyl,-cetylstearyl, or-oleoyl ether, as in polyhydroxyethylene4 or 6 or 8 or 10 or 12, etc.,-lauryl ether (as in Brij series), or in the corresponding ester, e. g. of polyhydroxyethylen-8-stearate (Myrj 45),laurate or-oleate type, or in polyethoxylated castor oil 40, a sorbitanemonoalkylate (e.g. in Arlacel or Span), esp. sorbitane-monolaurate, an acylor alkanoyl-N-methylglucamide, esp. in or decanoyl-or dodecanoyl-Nmethylglucamide, an alkyl-sulphate (salt), e. g. in lauryl-or oleoyl-sulphate, sodium deoxycholate, sodium glycodeoxycholate, sodium oleate, sodium taurate, a fatty acid salt, such as sodium elaidate, sodium linoleate, sodium laurate, a lysophospholipid, such as n-octadecylene (=oleoyl)glycerophosphatidic acid,-phosphorylglycerol, or-phosphorylserine, nacyl-, e. g. lauryl or oleoyl-glycero-phosphatidic acid,-phosphorylglycorol, or-phosphorylserine, n-tetradecyl-glycero-phosphatidic acid,phosphorylglycerol, or-phosphorylserine, a corresponding palmitoeloyl-, elaidoyl-, vaccenyl-lysophospholipid or a corresponding short-chain phospholipid, or else a surface-active polypeptide. Numerous other examples of edge active substances, of which many can be considered pharmaceutically acceptable, are given in US 6,165,500 which is hereby incorporated by reference.

As will be recognised by those skilled in the type of disease or condition to be treated as well as the type of skin, will set the specific requirement with respect to relative ratio between the constituents. The ratio between the lipid and the edge active substance(s) is generally from about 50:1 to about 1:500, such as from about 5:1 to about 1:500, from 20:1 to 1:500, from 10:1 to 1:500, from 10:1 to 1:200, from 10:1 to 1:100, from 5:1 to 1:200, from 5:1 to 1:100, from 5:1 to 1:50, from 5:1 to 1:20, or from 5:1 to 1:10, preferably from 5:1 to 1:5.

"Liposomes" are known as self-assembling structures comprising one or more lipid bilayers, each of which surrounds an aqueous compartment and comprises two opposing monolayers of amphiphathic lipid molecules. Amphiphathic lipids (herein i.a. lipid derivatives) comprise a polar (hydrophilic) headgroup region (corresponding to the substituent R³ in the lipid derivatives) covalently linked to one or two non-polar (hydrophobic) aliphatic groups (corresponding to R¹ and R² in the lipid derivatives). Energetically unfavourable contacts between the hydrophobic groups and the aqueous medium are generally believed to induce lipid molecules to rearrange such that the polar headgroups are oriented towards the aqueous medium while the hydrophobic groups reorient towards the interior of the bilayer. An energetically stable structure is formed in which the hydrophobic groups are effectively shielded from coming into contact with the aqueous medium.

Liposomes can have a single lipid bilayer (unilamellar liposomes, "ULVs"), or multiple lipid bilayers (multilamellar liposomes, "MLVs"), and can be made by a variety of methods (for a review, see, for example, Deamer and Uster, Liposomes, Marcel Dekker, N.Y., 1983, 27-52). These methods include Bangham's methods for making multilamellar liposomes (MLVs); Lenk's, Fountain's and Cullis' methods for making MLVs with substantially equal interlamellar solute distribution (see, e.g., US 4,522,803, US 4,588,578, US 5,030,453, US 5,169,637 and US 4,975,282); and Papahadjopoulos et al.'s reverse-phase evaporation method (US 4,235,871) for preparing oligolamellar liposomes. ULVs can be produced from MLVs by such methods as sonication (see Papahadjopoulos et al., Biochem. Biophys. Acta, 135, 624 (1968)) or extrusion (US 5,008,050 and US 5,059,421). The liposome can be produced by the methods of any of these disclosures as well as by the method used in US 6,165,500, the contents of all are incorporated herein by reference.

Various methodologies, such as sonication, homogenisation, French Press application and milling can be used to prepare liposomes of a smaller size from larger liposomes. Extrusion (see US 5,008,050) can be used to size reduce liposomes, that is to produce liposomes having a predetermined mean size by forcing the liposomes, under pressure, through filter pores of a defined, selected size. Tangential flow filtration (see WO 89/08846), can also be used to regularise the size of liposomes, that is, to produce liposomes having a population of liposomes having less size heterogeneity, and a more homogeneous, defined size distribution. The contents of these documents are incorporated herein by reference. Liposome sizes can also be determined by a number of techniques, such as quasi-electric light scattering, and with equipment, e.g., Nicomp^{®} particle sizers, well within the possession of ordinarily skilled artisans.

It is quite interesting to note that the lipid derivatives together with the edge active substance(s) constitute the major part of the lipid-based system (liposome system). This fact resides in the structural (but not functional) similarity between the lipid derivatives and lipids. Thus, it is believed that the lipid derivatives and edge active substances can be the sole constituent of liposomes, i.e. up to 100 mol% of the total dehydrated liposomes can be constituted by the lipid derivatives and edge active substances. This is in contrast to the known mono-ether lysolipids, which can only constitute a minor part of the liposomes.

This being said, it is believed that the combination of lipid derivatives and edge active substance will typically constitute from 50-100 mol%, such as 60-100 mol%, preferably from 75-100 mol%, in particular 90-100 mol%, based on the total dehydrated liposome.

Liposomes may also include other constituents which may or may not have a pharmaceutical effect, but which will render the liposome structure more acceptable for the topical applications of the skin. Examples hereof are other lipids, sterolic compounds, and targeting compounds, etc. In some interesting embodiments, the liposomes also comprises other lipids, e.g. diacylphospholipids.

The liposomes comprising lipid derivatives may (in principle) exclusively consist of the lipid derivatives. However, in order to modify the liposomes, "other lipids" may be included as well. Other lipids are selected for their ability to adapt compatible packing conformations with the lipid derivative components of the bilayer such that the all the lipid constituents are tightly packed, and release of the lipid derivatives from the bilayer is inhibited. Lipid-based factors contributing to compatible packing conformations are well known to ordinarily skilled artisans and include, without limitation, acyl chain length and degree of unsaturation, as well as the headgroup size and charge. Accordingly, suitable other lipids, including various phosphatidylethanolamines ("PE's") such as egg or soya phospholipids or dioleoyl phosphatidylethanolamine ("DOPE"), can be selected by ordinarily skilled artisans without undue experimentation. Lipids may be modified in various way, e.g. by headgroup derivatisation with dicarboxylic acids, such as glutaric, sebacic, succinic and tartaric acids, preferably the dicarboxylic acid is glutaric acid ("GA").

Accordingly, suitable headgroup-derivatised lipids include phosphatidylethanolamine-dicarboxylic acids such as dipalmitoyl phosphatidylethanolamine-glutaric acid ("DPPE-GA"), palmitoyloleoyl phosphatidylethanolamine-glutaric acid ("POPE-GA") and dioleoyl phosphatidylethanolamine-glutaric acid ("DOPE-GA"). Most preferably, the derivatised lipid is DOPE-GA.

The total content of "other lipids" will typically be in the range of 0-30 mol%, in particular 1-10 mol% or 0-10 mol%, based on the total dehydrated liposome.

Sterolic compound included in the liposome may generally affects the fluidity of lipid bilayers. Accordingly, sterol interactions with surrounding hydrocarbon groups generally inhibit emigration of these groups from the bilayer. An examples of a sterolic compound (sterol) to be included in the liposome is cholesterol, but a variety of other sterolic compounds are possible. It is generally believed that the content of sterolic compound, if present, will be in the range of 0-25 mol%, in particular 0-10 mol%, such as 0-5 mol%, based on the total dehydrated liposome.

Still other ingredients may constitute 0-2 mol%, in particular 0-1 mol%, based on the total dehydrated liposome.

Lipid-based particulate systems, i.e. liposomes; of sizes covering a broad range may be prepared according to the above-mentioned techniques. Depending on the particular application, suitable sizes for pharmaceutical applications will normally be in the range of 20-10,000 nm, in particular in the range of 30-1000 nm. Sizes in the range of 50-200 nm are normally preferred.

The liposomes can be unilamellar or multilamellar. Some preferred liposomes are unilamellar and have diameters of less than about 200 nm, more preferably, from greater than about 50 nm to less than about 200 nm.

The liposomes are typically - as known in the art - prepared by a method comprising the steps of: (a) dissolving the lipid derivative and edge active substances and other constituents in an organic solvent; (b) removing the organic solvent from the lipid derivative solution of step (a); and (c) hydrating the product of step (b) with an aqueous solvent so as to form liposomes.

The method may further comprise a step of adding an second drug substance (see below) to the organic solvent of step (a) or the aqueous phase of step (c).

Subsequently, the method may comprise a step of extruding the liposomes produced in step (c) through a filter to produce liposomes of a certain size, e.g. 100 nm.

The liposome can be dehydrated, stored and then reconstituted such that a substantial portion of its internal contents is retained. Liposomal dehydration generally requires use of a hydrophilic drying protectant such as a disaccharide sugar at both the inside and outside surfaces of the liposome bilayers (see US 4,880,635). This hydrophilic compound is generally believed to prevent the rearrangement of the lipids in the liposome, so that the size and contents are maintained during the drying procedure and through subsequent rehydration. Appropriate qualities for such drying protectants are that they are strong hydrogen bond acceptors, and possess stereochemical features that preserve the intramolecular spacing of the liposome bilayer components. Alternatively, the drying protectant can be omitted if the liposome preparation is not frozen prior to dehydration, and sufficient water remains in the preparation subsequent to dehydration.

### Lipid derivative liposomes as drug carrier systems

As mentioned above, the liposomes including the lipid derivatives may also include second drug substances. In a particular embodiment, the lipid-based drug delivery system described above is in the form of liposomes wherein a second drug substance is incorporated. It should be understood that second drug substances may comprise pharmaceutically active ingredients which may have an individual or synergistic pharmaceutical effect in combination with the lipid derivative and lysolipid derivatives. The term "second" does not necessarily imply that the pharmaceutical effect of the second drug substance is inferior in relation to that of, e.g., the active drug substance derived from the prodrug, but is merely used to differentiate between the two groups of substances.

A possible "second drug substance" is any compound or composition of matter that can be administered to mammals, preferably humans. Such agents can have biological activity in mammals. Second drug substances which may be associated with liposomes include, but are not limited to: antiviral agents such as acyclovir, zidovudine and the interferons; antibacterial agents such as aminoglycosides, cephalosporins and tetracyclines; antifungal agents such as polyene antibiotics, imidazoles and triazoles; antimetabolic agents such as folic acid, and purine and pyrimidine analogs; antineoplastic agents such as the anthracycline antibiotics and plant alkaloids; sterols such as cholesterol; carbohydrates, e.g., sugars and starches; amino acids, peptides, proteins such as cell receptor proteins, immunoglobulins, enzymes, hormones, neurotransmitters and glycoproteins; dyes; local anesthetics; and the like.

Liposomes can be loaded with one or more second drug substances by solubilising the drug in the lipid or aqueous phase used to prepare the liposomes. Alternatively, ionisable second drug substances can be loaded into liposomes by first forming the liposomes, establishing an electrochemical potential, e.g., by way of a pH gradient, across the outermost liposomal bilayer, and then adding the ionisable second drug substance to the aqueous medium external to the liposome (see, e.g., US 5,077,056 and WO 86/01102).

Methods of preparing lipophilic drug derivatives which are suitable for liposome formulation are known in the art (see e.g., US 5,534,499 and US 6,118,011 describing covalent attachment of therapeutic agents to a fatty acid chain of a phospholipid). A micellar formulation of taxol is described in Alkan-Onkyuksel et al., Pharmaceutical Research, 11:206 (1994).

Accordingly, the second drug substance may be any of a wide variety of known and possible pharmaceutically active ingredients applicable for diseases and conditions in epidermal-, dermal or subcutaneous tissue of the skin. Due to the mechanism involved in the degradation of the liposomes, it is preferred that the second drug substance is one relating to skin diseases and/or conditions associated with a localised increase in extracellular PLA₂ activity such as: arachidonoyl derivatives.

It is envisaged that the second drug substance will be distributed in the liposomes according to their hydrophilicity, i.e. hydrophilic second drug substances will tend to be present in the cavity of the liposomes and hydrophobic second drug substances will tend to be present in the hydrophobic bilayer. Method for incorporation of second drug substances are know in the art as has been made clear above.

It should be understood from the above, that the lipid derivatives may - as prodrugs or discrete constituents - posses a pharmaceutical activity. However, in a particular embodiment, the present invention furthermore relates to the use of a lipid based drug delivery system in the form of a liposome for administration of an second drug substance, wherein the second drug substance is incorporated in the system (e.g. where the second drug substance is encapsulated in the interior of the liposome or in the membrane part of the liposome), said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular PLA₂ to the extent that the organic radical can be hydrolytically cleaved off, whereas the aliphatic group remains substantially unaffected, so as to result in an organic acid fragment or an organic alcohol fragment and a lysolipid fragment, said lysolipid fragment not being a substrate for lysophospholipase.

As above for the system according to the other embodiment, the organic radical which can be hydrolytically cleaved off, may be an auxiliary drug substance or an efficiency modifier for the second drug substance. It should be understood that the lipid derivative is a lipid derivative as defined further above. Typically, the combination of the lipid derivative and the edge active substance constitutes 50-100 mol%, such as 90-100 mol%, of the total dehydrated (liposome) system.

### Pharmaceutical preparations and therapeutic uses

The diseases or conditions to be treated or alleviated are typically selected from skin cancer, psoriasis, and inflammatory conditions.

The pharmaceutical composition to be administered to the person in need therefor typically comprises a pharmaceutically acceptable carrier and the liposome.

"Pharmaceutically acceptable carriers" as used herein are those media generally acceptable for use in connection with the administration of liposomes, including liposomal drug formulations, to mammals, including humans. Pharmaceutically acceptable carriers are generally formulated according to a number of factors well within the purview of the ordinarily skilled artisan to determine and account for, including without limitation: the particular active drug substance and/or second drug substance used, the liposome preparation, its concentration, stability and intended bioavailability; the disease, disorder or condition being treated with the liposomal composition; the subject, its age, size and general condition; and the composition's intended route of administration (here: dermal). Pharmaceutically acceptable carriers can contain additional ingredients, for example those which enhance the stability of the active ingredients included, such as preservatives and anti-oxidants.

The topical compositions of the present invention may be made into a wide variety of product types. These include, but are not limited to solutions, lotions, creams, beach products, gels, sticks, sprays, pads, ointments, pastes, mousses and cosmetics. These product types may comprise several types of liposome carrier systems including the substances described in the present invention.

The formulation and preparation of such compositions is well-known to those skilled in the art of pharmaceutical formulation. Specific formulations can be found in the textbook entitled "Remington's Pharmaceutical Sciences".

In dermatology, application doses of up to 50 mg, often up to 10 mg and very frequently less than 2.5 mg (or even less than 1 mg) of carrier substance are used per square cm of skin surface, the given masses pertaining to the basic carrier substance. The optimal mass depends on the carrier composition, desired penetration depth and duration of action, as well as on the detailed application site.

For dermal applications, preferably particles or vesicles with a diameter of the order of 100-10,000 nm, frequently in the range of 100 to 400 nm, and most frequently with sizes between 100 and 200 nm are used as carriers.

The invention will be illustrated by the following non-limiting examples.

### EXAMPLES

### Example 1

### Liposome preparation

Unilamellar fully hydrated liposomes with a narrow size distribution were made from 1-O-hexadecyl-2-hexadecanoyl-*sn*-glycero-3-phosphocholine (1-O-DPPC) and di-hexadecanoyl-*sn*-glycero-3-phosphocholine (DPPC). DPPC were obtained from Avanti Polar lipids and 1-O-DPPC were synthesised in our laboratory. Briefly, weighed amounts of DPPC or 1-O-DPPC were dissolved in chloroform. The solvent was removed by a gentle stream of N₂ and the lipid films were dried overnight under low pressure to remove trace amounts of solvent. Multilamellar vesicles were made by dispersing the dried lipids in a buffer solution containing: 150 mM KCL, 10 mM HEPES (pH = 7.5), 1 mM NaN₃, 30 µM CaCl₂ and 10 µM EDTA. The multilamellar vesicles were extruded ten times through two stacked 100 nm pore size polycarbonate filters as described by Mayer et al., *Biochim. Biophys. Acta,* **858,** 161-168.

Heat capacity curves were obtained using a N-DSC II differential scanning calorimetor (Calorimetry Sciences Corp., Provo) of the power compensating type with a cell volume of 0.34 mL. Before scanning, the liposome suspension was equilibrated for 50 min in the calorimeter at the starting temperature. A scan rate of +10 °C/h was used. The lipid concentration was 1 mM. The gel-to-fluid transition of the multilamellar liposomes (MLV) is characterised as a sharp first-order transition, as reflected by the narrow peak in the heat capacity curves shown in Figs. 1a and 1 b (upper curves) for 1-O-DPPC and DPPC. The sharp peak reflects the transitional behaviour of multilamellar liposomes and is in contrast to the broader gel-to-fluid transition observed for unilamellar liposomes (LUV) (Pedersen et al., 1996, *Biophys. J*. **71**, 554-560) as shown in Figs. 1a and 1b (lower curves) for the unilamellar extruded 1-O-DPPC and DPPC liposomes.

### Example 2

Phospholipase A₂ reaction profile and lag time measurements Purified snake-venom phospholipase A₂ (PLA₂ from *Agikistrodon piscivorus piscivorus*) has been isolated according to the procedure of Maraganore et al., J. *Biol. Chem.* **259,** 13839-13843. This PLA₂ enzyme belongs to the class of low-molecular weight 14kD secretory enzymes which display structural similarity to human extracellular phospholipase A₂ indicating a common molecular mechanisms of the phospholipase catalysed hydrolysis at the lipid-membrane interface (Wery et al., *Nature* **352,** 79-82; Hønger et al. *Biochemistry* **35,** 9003-9006; Vermehren et al., *Biochimica et Biophysica Acta* **1373,** 27-36). Unilamellar fully hydrated liposomes with a narrow size distribution were prepared from 1-O-hexadecyl-2-hexadecanoyl-*sn*-glycero-3-phosphocholine (1-O-DPPC) and from 1-O-DPPC with 5 mol% 1-O-hexadecyl-2-hexadecanoyl-*sn*-glycero-3-phosphoethanolamine-N-[methoxy(polyethylene glycol)-350] (1-O-DPPE-PEG350). Assay conditions for the PLA₂ reaction time profile shown in Fig. 2 and the lag-time and percent hydrolysis reported in Table 1 were: 0.15 mM unilamellar liposomes, 150 nM PLA₂, 150 mM KCL, 10 mM HEPES (pH 7.5), 1 mM NaN₃, 30 µM CaCl₂, and 10 µM EDTA.

**Table 1. Lag-time and percent hydrolysed 1-O-DPPC at 41°C as determined by HPLC. The lipid concentration was 0.150 mM in a 10 mM HEPES-buffer (pH = 7.5).**

| Composition | Lag-time (sec) | 1-O-DPPC (%) |
|---|---|---|
| 100% 1-O-DPPC | 583 | 79 |
| 95% 1-O-DPPC/ 5 % 1-O-DPPE-PEG350 | 128 | 73 |

The catalytic reaction was initiated by adding 8.9 µL of a 42 µM PLA₂ (150 nM) stock solution to 2.5 ml of the thermostated liposome suspension (0.150 mM) equilibrated for 800 sec prior to addition of PLA₂. The characteristic lag-burst behaviour of PLA₂ towards the liposomes is signalled by a sudden increase in the intrinsic fluorescence from PLA₂ at 340 nm after excitation at 285 nm followed by a concomitant decrease in the 90° light scattering from the lipid suspension (Hønger et al., *Biochemistry* **35**, 9003-9006). Samples for HPLC analysis of the amount of non-hydrolysed 1-O-DPPC remaining and consequently the amount of 1-O-hexadecyl-2-hydroxy-*sn*-glycero-3-phosphocholine (lyso-1-O-PPC) generated were taken before adding PLA₂ and 1200 sec after the observed lag-time. 100 µl aliquots were withdrawn from the lipid suspension and rapidly mixed with 1 ml chloroform/methanol/acetic acid (2:4:1) solution in order to quench the enzymatic reaction. The solution was washed with 1 ml of water and 20 µl of the heavy organic phase was used for HPLC. The HPLC chromatograms in Fig. 3 show the amounts of 1-O-DPPC before and after (t = 3000 sec) the addition of PLA₂ (t = 800 sec) to the liposome suspension. HPLC analysis was made using a 5 µm diol column, a mobile phase composed of chloroform/methanol/water (730:230:30, v/v) and an evaporative light scattering detector. The turnover of the PLA₂ catalysed lipid hydrolysis of 1-O-DPPC to lyso-1-O-PPC was measured by HPLC (see Table 1). The intrinsic enzyme fluorescence and 90° light scattering were measured as a function of time as shown in Fig. 2.

### Example 3

### Phospholipase A₂ induced release of an incapsulated water-soluble model drug

Multilamellar 1-O-DPPC-liposomes were made in the presence of fluorescent calcein in a self-quenching concentration of 20 mM by hydrating a film of 1-O-hexadecyl-2-hexadecanoyl-*sn*-glycero-3-phosphocholine in a HEPES buffer solution at pH=7.5 for one hour at 10°C above the phase transition temperature. Unilamellar liposomes were formed by extruding the multilamellar liposomes ten times through two stacked 100 nm polycarbonate filters. The unilamellar liposomes were rapidly cooled to a temperature below the transition temperature, and the calcein-containing 1-O-DPPC liposomes were separated from free calcein using a chromatographic column packed with Sephadex G-50.

Assay conditions for the PLA₂ induced calcein release were 25 µM unilamellar 1-O-DPPC-liposomes, 25 nM PLA₂, 150 mM KCL, 10 mM HEPES (pH 7.5 or 8.0), 1 mM NaN₃, 30 µM CaCl₂, and 10 µM EDTA. PLA₂ was added at time 900 sec to 2.5 ml of the thermostated 1-O-DPPC-liposome suspension equilibrated for at least 20 min at 37°C prior to addition of PLA₂. The percentage of calcein released is determined as: % Release = 100 x (I_{F(t)} - I_{B})/(I_{T} - I_{B}), where I_{F(t)} is the measured fluorescence at time t after addition of the enzyme, I_{B} is the background fluorescence, and I_{T} is the total fluorescence measured after addition of Triton X-100 which leads to complete release of calcein by breaking up the 1-O-DPPC-liposomes. PLA₂ induced at total release of 90 percent of the entrapped calcein in the 1-O-DPPC-liposomes as shown in Fig. 4.

### Example 4

### Phospholipase A₂ controlled permeability increase of a target model membrane

Multilamellar model membrane target liposomes were made in the presence of fluorescent calcein in a self-quenching concentration of 20 mM by hydrating a film of 1,2-O-dioctadecyl-*sn*-glycero-3-phosphatidylcholines (D-O-SPC) in a HEPES buffer solution at pH=7.5 for one hour at 10°C above the phase transition temperature (Tₘ=55°C). Unilamellar liposomes were made by extruding the multilamellar target liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50. The unilamellar carrier liposomes composed of 1-O-hexadecyl-2-hexadecanoyl-*sn*-glycero-3-phosphocholine were prepared as described above. Calcein release from the target liposomes is determined by measuring the fluorescent intensity at 520 nm after excitation at 492 nm.

The concentrations of D-O-SPC and 1-O-DPPC-liposomes were 25 µM. Snake venom PLA₂ (*Agkistrodon piscivorus piscivorus*) was added (25 nM) to initiate the hydrolytic reaction leading to the formation of 1-O-hexadecyl-2-hydroxy-*sn*-glycero-3-phosphocholine (lyso-1-O-DPPC) and fatty acid hydrolysis products. As calcein is released from the D-O-SPC liposomes, due to the incorporation of the non-bilayer forming polymer-lyso-1-O-DPPC and fatty acid hydrolysis products into the target lipid membrane, a linear increase in the fluorescence at 520 nm after excitation at 492 nm is observed when calcein is diluted into the surrounding buffer medium as shown in Fig. 5. The percentage of calcein released is determined as described above (see Example 3).

### Example 5

### Enhancement of phospholipase A2 activity by negatively charged polymer grafted 1-O-lipids

Unilamellar fully hydrated liposomes with a narrow size distribution were prepared from 1-O-hexadecyl-2-hexadecanoyl-*sn*-glycero-3-phosphocholine (1-O-DPPC) and 1-O-DPPC with 5 mol% 1-O-hexadecyl-2-hexadecanoyl-*sn*-glycero-3-phosphoethanolamine-N-[methoxy(polyethylene glycol)-350] (1-O-DPPE-PEG350), as described in example 2. Assay conditions for the PLA₂ lag-time measurements were 0.15 mM unilamellar liposomes, 150 nM PLA₂, 150 mM KCL, 10 mM HEPES (pH 7.5), 1 mM NaN₃, 30 µM CaCl₂, and 10 µM EDTA. The catalytic reaction was initiated by adding 8.9 µL of a 42 µM PLA₂ stock solution to 2.5 ml of the thermostated liposomes suspension equilibrated for 800 seconds at 41°C prior to addition of PLA₂. The time elapsed before the onset of rapid enzymatic activity is determined by a sudden increase in the intrinsic fluorescence from PLA₂ at 340 nm after excitation at 285 nm. The results shown in Fig. 7 show a significant decrease in the lag time when 5 mol% of the negatively charged 1-O-DPPE-PEG₃₅₀ is incorporated into the 1-O-DPPC liposomes.

### Example 6

### Preparation of micelles composed of 1-O-DPPE-PEG350 and DSPE-PEG750.

Micelles were made from 1-O-hexadecyl-2-hexadecanoyl-*sn*-glycero-3-phosphoethanolamine-N-[methoxy(polyethylene glycol)-350] (1-O-DPPE-PEG350), di-octadecanoyl-sn-glycero-3-phosphoethanolamine-N-[methoxy(polyethylene glycol)-750 (DSPE-PEG750). Briefly, weighed amounts of the polymer-lipid were dissolved in chloroform. The solvent was removed by a gentle stream of N₂. The lipid films were then dried overnight under low pressure to remove trace amounts of solvent. Micelles were made by dispersing the dried polymer-lipids in a buffer solution containing: 150 mM KCL, 10 mM HEPES (pH = 7.5), 1 mM NaN₃, 30 µM CaCl₂ and 10 µM EDTA.

### Example 7

### Permeability increase of a target model membranes controlled by phospholipase A₂ hydrolysis of micelles

Multilamellar model membrane target liposomes were made in the presence of fluorescent calcein in a self-quenching concentration of 20 mM by hydrating a film of 1,2-O-dioctadecyl-*sn*-glycero-3-phosphatidylcholines (D-O-SPC) in a HEPES buffer solution at pH=7.5 for one hour at 10°C above the phase transition temperature (Tₘ=55°C). Unilamellar liposomes were made by extruding the multilamellar liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50. Micelles composed of 1-O-hexadecyl-2-hexadecanoyl-*sn*-glycero-3-phosphoethanolamine-N-[methoxy(polyethylene glycol)-350] (1-O-DPPE-PEG350), di-octadecanoyl-*sn*-glycero-3-phosphoethanolamine-N-[methoxy(polyethylene glycol)-750 (DSPE-PEG750). were prepared as described in example 6. Calcein release from the target liposomes is determined by measuring the fluorescent intensity at 520 nm after excitation at 492 nm.

The concentrations of D-O-SPC and polymer-lipid micelles were 25 µM. Snake venom PLA₂ (*Agkistrodon piscivorus piscivorus*) was added (25 nM) to initiate the hydrolytic reaction leading to instant formation of the polymer-lyso-1-O-DPPE and the free fatty acid hydrolysis products. As calcein is released from the D-O-SPC liposomes, due to the incorporation of the non-bilayer forming polymer-lyso-1-O-lipids and fatty acid into the target lipid membrane, a linear increase in the fluorescence at 520 nm after excitation at 492 nm is observed when calcein is diluted into the surrounding buffer medium as shown in Fig. 7. The percentage of calcein released is determined as described in example 3. PLA₂ catalysed hydrolysis of 1-O-DPPE-PEG350 induced the fastest release rate.

### Example 8

### Hydrolysis of micelles composed of DSPE-PEG750

The hydrolysis of micelles composed DSPE-PEG750 was followed by analysis of the amount of stearic acid generated. The catalytic reaction was initiated by adding 8.9 µL of a 42 µM PLA₂ (150 nM) stock solution to 2.5 ml a thermostated micelle suspension of DSPE-PEG750 (0.150 mM) equilibrated at 45 °C for 600 seconds prior to addition of PLA₂. The characteristic burst behaviour of PLA₂ towards the micelles is signalled by a sudden increase in the intrinsic fluorescence from PLA₂ at 340 nm after excitation at 285 nm followed by a concomitant decrease in the 90° light scattering from the lipid suspension (Hønger et al., *Biochemistry* **35**, 9003-9006). Samples for HPLC analysis of the amount of stearic acid generated were taken before adding PLA₂ and 100 sec after the observed lag-time. The HPLC chromatograms in Fig. 8 shows the amount of stearic acid generated 100 sec after the observed lag time (10 sec) at 45°C. The amount (0.156 mM) of stearic acid generated by hydrolysis was equal to 100% hydrolysis of the DSPE-PEG750 polymer-lipids. HPLC analysis was made using a 5 µm diol column, a mobile phase composed of chloroform/methanol/water (730:230:30, v/v) and an evaporative light scattering detector (see example 2).

### Example 9

### Model examples

Liposomes composed of neutral or negatively charged phospholipids can act as flexible dermal drug- and prodrug-delivery systems targeting a number of different skin diseases that are associated with increased phospholipase A2 levels at the diseased site in the skin. The flexible liposomes will when applied on the surface of the skin begin to migrate into the skin where they eventually will reach the diseased subcutaneous target tissue. In the examples herein are described an experimental model system illustrating the principle for improved drug and prodrug-delivery to the skin which takes advantage of an elevated activity of extracelluar phospholipase A₂ in the diseased skin. The phospholipase A₂ hydrolyses the lipid-based proenhancer in the flexible carrier liposome, producing lyso-phospholipid and free fatty acid, which are shown in a synergistic way to lead to enhanced liposome destabilisation and drug release at the same time as the permeability of the target membrane is enhanced. The proposed system can be made thermosensitive and offers a rational way for developing smart liposome-based drug delivery systems by incorporating into the carrier specific lipid-based proenhancers, prodestabilisers or prodrugs that automatically become activated by phospholipase A₂ only at the infected target sites.

Liposomes are self-assembled lipid systems and their stability is therefore to a large extent controlled by non-specific physical interactions. Insight into the molecular control of the physical properties of liposomes is therefore important for manipulating and tailoring the liposomal properties in relation to specific drug-delivery purposes. As an example, the thermally induced gel-fluid lipid phase transition has been exploited and optimised design systems for enhanced release of drugs due to hyperthermia. It would be desirable if an intelligent and versatile drug-delivery system could be designed which has built in a dual virtual trigger mechanism of simultaneous (i) enhanced drug release selectively in the liver and spleen (the target tissue) and (ii) enhanced transport of the drug into the infected cells. This principle is illustrated schematically in Fig. 9.

By the examples herein is described the development of a simple and operative experimental biophysical model system which sustains such a dual mechanism to be triggered in the infected subcutaneous target tissue. The model assumes elevated activity of extracellular phospholipase A₂ in the diseased skin target tissue, as is the case in diseased tissue where the level of extracellular PLA₂ can be manifold magnified. Upon exposure to extracellular PLA₂, the phospholipids of e.g. negatively charged liposomes have been shown to suffer enhanced hydrolysis compared to neutral liposomes. This leads to destabilisation of the liposome and enhanced release of the encapsulated drug. The hydrolysis products, lyso-phospholipids and free fatty acids, act in turn as absorption enhancers for drug permeation across the target membrane. In this way the phospholipids of the carrier liposome behave as prodestabilisers at the site of the carrier and as proenhancers at the site of the target membrane. Molecular details of this principle are illustrated schematically in Fig. 10.

The experimental model system consists of a negatively charged liposome carrier and a model target membrane. The carrier is a 100 nm unilamellar liposome made of dipalmitoyl phosphatidylcholine lipids (DPPC) with small amounts (2.5 mol%) of negatively charged lipid of the type dipalmitoyl phosphatidylethanolamine (DPPE)-PEG₂₀₀₀. The target membrane is another liposome made of 1,2-O-dioctadecyl-sn-glycero-phosphatidylcholine (D-O-SPC) which is a phospholipid where the acyl linkages of the stearoyl chains are ether bonds. In contrast to DPPC, D-O-SPC is inert towards PLA₂-catalysed hydrolysis thereby mimicking the stability of an intact target cell membrane toward degradation by its own enzymes. This experimental assay, which permits simultaneous as well as separate investigation of the effect of destabilisers at the carrier liposomes and the effect of enhancers at the target membrane, involves entrapment of a water-soluble fluorescent calcein model drug in a self-quenching concentration, in the interior of the non-hydrolysable target liposome, rather than in the carrier liposome. The enhanced level of extracellular PLA₂ at the target membrane can then be simulated by adding extracellular PLA₂ to initiate the hydrolytic reaction in a suspension of the carrier and target liposomes. The permeation of calcein across the D-O-SPC target membrane is subsequently monitored by the increase in fluorescence. In order to investigate the effect of the presence of small amounts of negatively charged lipids in the carrier liposome, a similar experiment was performed with conventional bare DPPC liposomes. Furthermore, in order to compare and discriminate the permeability enhancing effect of lyso-phospholipids from that of free fatty acids, experiments without enzymes were carried out where lyso-phospholipids and free fatty acids were added simultaneously or separately to the target liposomes.

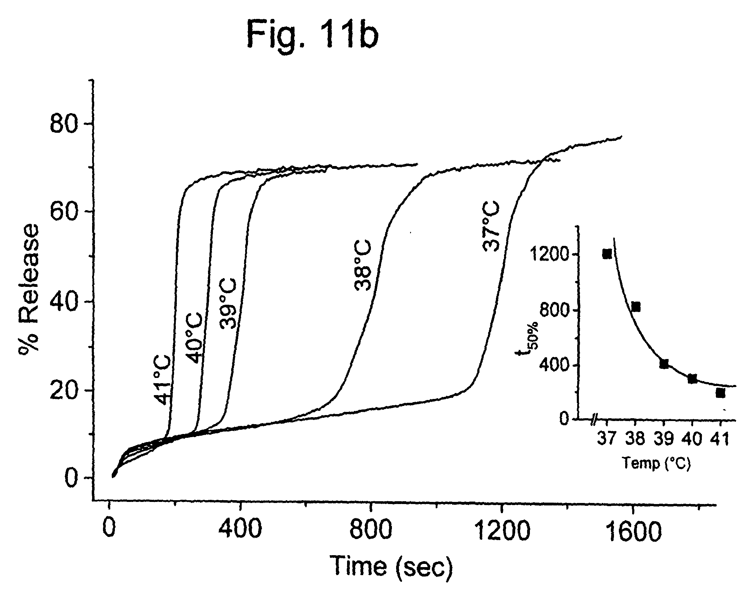

In Fig. 11.a are shown the results for the release of calcein as a function of time after adding PLA₂ to the system. The reaction time-course of the particular PLA₂ used has a characteristic lag-burst behaviour with a so-called lag time which conveniently can be used as a measure of the enzymatic activity. A dramatic decrease in the lag time and a concomitant enhancement of the rate of release are observed when the carrier liposomes contain the negativly charged, DPPE-PEG₂₀₀₀, in accordance with previous findings of enhanced extracellular PLA₂ degradation of negatively charged polymer-coated liposomes.

These results suggest that the products of the PLA₂-catalysed hydrolysis of the DPPC liposomal carrier, lyso-phospholipid and free fatty acid, which are produced in a 1:1 mixture, are incorporated into the target membrane, leading to a large increase in membrane permeability. These products, which have very low water solubility, are known, due to their non-cylindrical molecular shapes, to induce a curvature stress field in the membrane or small-scale lateral phase separation which induce membrane defects and increased permeability. This is substantiated by the data in Fig. 12 which show that the addition of lyso-phospholipid or fatty acid separately to the present target system, in the absence of PLA₂, leads to an increased rate of calcein release across the target membrane. However, the crucial finding is that if lyso-phospholipid and free fatty acid are added simultaneously in a 1:1 mixture, a dramatic enhancement in the rate of release is observed as shown in Fig. 12. This strongly suggests that the two enhancers act in a synergistic fashion, thereby highlighting the unique possibility in exploiting PLA₂-catalysed hydrolysis for combined destabilisation of the carrier liposome and enhancement of drug transport across the target membrane. The synergistic effect is further augmented by the fact that extracellular PLA₂ is activated by its own hydrolysis products revealing the degradable phospholipids of the carrier liposome as a kind of proactivators.

It should be pointed out that the effect in the present drug-delivery model system of using lipids as proenhancers and prodestabilisers via extracellular PLA₂ activity is dynamic and refers to an intrinsic time scale. This time scale is the effective retention time of the carrier liposomes near the target membrane. The more rapidly the enzyme becomes active, the faster is the drug release and the larger the drug absorption during the time which the carrier spends near the target. Furthermore, the faster the enzyme works the more readily it becomes available for hydrolysis of other drug-carrying liposomes that approach the diseased target site. Once it has been established that extracellular PLA₂ activity can be used to control drug release, several rational ways open up for intelligent improvements of the proposed drug-delivery system via use of well-known mechanisms of altering extracellular PLA₂ activity by manipulating the physical properties of the lipid bilayer to which the enzyme is known to be sensitive. Hence the strategy is to modify certain physical properties of the carrier liposomes without significantly changing their vascular circulation time. We shall illustrate this general principle by demonstrating the effects of both a physico-chemical factor, the lipid composition of the carrier, and an enviromental (thermodynamic) factor, the local temperature at the target site.

Short-chain phospholipids, such as didecanoyl phosphatidylcholine (DCPC), activate extracellular PLA₂. The effect on calcein permeation across the target membranes induced by incorporation of a small amount of DCPC into the carrier PEG-liposomes is also shown in Fig. 11.a. The release is very fast due to an almost instantaneous activation of the enzyme. We have furthermore found that extracellular PLA₂ becomes deactivated (data not shown) when a large amount of cholesterol (≈20 mol %) is incorporated into liposomes. In contrast we find that a small amount of cholesterol (≈3 mol%) activates extracellular PLA₂.

Temperature is known to have a dramatic and highly non-linear effect on extracellular PLA₂ activation in the region of the gel-fluid phase transition of saturated phospholipid bilayers. This effect is not caused by changes in the enzyme but by dramatic lateral structural changes in the lipid bilayer. It is possible to take advantage of this effect in the present drug-delivery system as suggested by the data in Fig. 11.b. As the temperature approaches the transition temperature at 41°C, the rate of calcein release is progressively enhanced as quantified by the time of 50% calcein release, t_{50%}, shown in the insert to Fig. 11.b. It has previously been suggested that hypertermia could be exploited to enhance drug release, and that local heating at predefined tumour areas could be used to locally destabilise drug-carrying liposomes, by exploiting the enhanced leakiness of liposomes at their phase transition. In the new model drug-delivery system proposed here, these thermosensitive possibilities are integrated and fully exploited via the thermal sensitivity of extracellular PLA₂ to the physical properties of the carrier liposome. In contrast to the case where the thermic effect can only be achieved by a local temperature increase using external heating sources at a predetermined tumour site of some minimal size, the PLA₂-controlled release will be enhanced everywhere where temperature and extracellular PLA₂ concentration are elevated, e.g. in inflammed tissue, independent of the size of the diseased region and without requiring a preceding localisation of the diseased tissue.

DPPC, DCPC, D-O-SPC, and DPPE-PEG₂₀₀₀ were obtained from Avanti Polar Lipids.. Purified snake venom PLA₂ (*Agkistrodon piscivorus piscivorus*) was a generous gift from dr. R. L. Biltonen. This PLA₂ enzyme belongs to the class of low-molecular weight, 14kD secretory enzymes which display structural similarity to human extracellular phospholipase A₂. Multilamellar target liposomes in the presence of fluorescent calcein in a self-quenching concentration of 20mM were made by hydrating a film of D-O-SPC in a HEPES buffer solution at pH=7.5 for one hour at 10°C above the phase transition temperature Tₘ=55°C. Unilamellar liposomes were made by extruding the multilamellar liposomes ten times through two stacked 100nm polycarbonate filters. The unilamellar liposomes were rapidly cooled to a temperature below the transition temperature, and the calcein-containing liposomes were separated from free calcein using a chromatographic column packed with Sephadex G-50. The unilamellar carrier liposomes of DPPC, DCPC and DPPE-PEG₂₀₀₀ were prepared in a similar fashion Tₘ=41°C). Calcein release from the target liposomes is determined by measuring the fluorescent intensity at 520nm after excitation at 492nm. All measurements are performed at temperatures where the lipids of both the carrier and target liposomes are in the gel state.

### Example 10

### Phospholipase A₂ concentration dependent release assay

Multilamellar 1-O-DPPC-liposomes with 10 mol% 1-O-DPPE-PEG350 were made in the presence of fluorescent calcein in a self-quenching concentration of 20 mM by hydrating a film of 90% 1-O-hexadecyl-2-hexadecanoyl-*sn*-glycero-3-phosphocholine and 10% 1-O-hexadecyl-2-hexadecanoyl-*sn*-glycero-3-phosphoethanolamine-N-[methoxy(polyethylene glycol)-350] in a HEPES buffer solution at pH=7.5 for one hour at 10 °C above the phase transition temperature. Unilamellar liposomes were formed by extruding the multilamellar liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50.

Assay conditions for the PLA₂ induced calcein release were 25 µM unilamellar liposomes, 50, 1 and 0.02 nM PLA₂, 150 mM KCL, 10 mM HEPES (pH 7.5), 1 mM NaN₃, 30 µM CaCl₂, and 10 µM EDTA. PLA₂ was added to 2.5 ml of the thermostated lipid suspension equilibrated for at least 300 sec at 35.5°C prior to addition of PLA₂. The percentage of calcein released is determined as: % Release = 100 x (I_{F(t)} - I_{B})/(I_{T} - I_{B}), where I_{F(t)} is the measured fluorescence at time t after addition of the enzyme, I_{B} is the background fluorescence, and I_{T} is the total fluorescence measured after addition of Triton X-100 which leads to complete release of calcein by breaking up the 1-O-DPPC-liposomes. Figure 13 show that the induced release of calcein was slowest when only 0.02 nM PLA₂ was added to the liposome suspension.

### Example 11

### Preparation of ultraflexible liposomes

Ultraflexible liposomes are prepared by dissolving 0.08- 0.32 mmol of a prodrug monoether lipid (1-O-lipid) mixture, of similar composition as found in soy phosphatidylcholine mixtures, in 80- 320 µl absolute ethanol and adding increasing amounts of oleic acid to create 18 samples with a molar lipid/surfactant ratio (US) starting at US = 0.4 and increasing by 0.2 units to US = 4. After thoroughly mixing of the lipids and surfactants, 5 ml 10 mM HEPES-buffer (pH=7.5- 8.0) is added to each of the lipid samples and the mixtures are incubated at 4 -10°C for 24 hours. The formed liposomes are extruded five times through two stacked 0.5 µm pore size polycarbonate filters as described by Mayer et al., *Biochim. Biophys. Acta,* **858,** 161-168 and then allowed to equilibrate 24 hours at 4- 10°C before measuring the size by dynamic light scattering using a Malver Master Sizer. The size of the vesicles is expected to be 300 - 400 nm and to be relatively independent of the US ratio.

The permeation resistance of the ultraflexible liposomes is determined by measuring the relative pressure needed to force the ultraflexible liposomes through a 0.2 µm polycarbonate filter. The relative pressure is expected to vary as a function of the lipid to surfactant ratio within the range of 1 to 10 MPa. Formulations with a US ratio of about 0.5 are expected to be almost perfectly permeable, with permeation resistance similar to that of pure water.

### Example 12

### Hydrolysis of negatively charged liposomes by phospholipase A2 in cell-free rat peritoneal fluid

Cell-free peritoneal fluid from rat with casein-induced acute inflammation was prepared by injecting 5 ml 1% sodium caseinate into the peritoneal cavity of a SRPD male rat, weighing 250- 260 g. The rat was sacrificed by bleeding after 24 hours and the inflammatory fluid was collected from the peritoneum and centrifuged at 1500 G for 20 min in order to obtain a cell-free peritoneal fluid.

Negatively charged fully hydrated unilamellar liposomes with a narrow size distribution were prepared from 89 mol% di-hexadecanoyl-*sn*-glycero-3-phosphoglycerol (DPPG), 10 mol% 1-O-hexadecyl-2-hexadecanoyl-*sn*-glycero-3-phosphoethanolamine-N-[methoxy(polyethylene glycol)-350] (1-O-DPPE-PEG350) and 1 mol% 1,2-bis-(1-pyrenedecanoyl) -*sn*-glycero-3-phosphocholine (bis-py-DPC). Bis-py-DPC is a PLA2 substrate with two adjacent pyrene fluorophores that form excited-state dimers (eximers) emitting at 470 nm upon excitation at 342 nm. Phospholipase catalysed hydrolysis separates the two fluorophores, which then emit at 380 nm (monomers).

Figure 14 shows the emission spectra obtained after excitation at 342 nm of bis-py-DPC incorporated in negatively charged liposomes (0.100 mM) before and after adding 100 nM PLA₂ (*Agkistrodon piscivorus piscivorus*). The observed changes in the emission spectrum after phospholipase mediated hydrolysis is used in a continuos assay, measuring the eximer emission at 470 nm simultaneously with the monomer emission at 380, upon excitation at 342 nm. Figure 15 shows the reaction time profile of rat phospholipase A₂ catalysed hydrolysis of the negatively charged liposomes. The catalytic reaction was initiated by adding cell-free peritoneal fluid to 2.5 ml of a thermostated liposome suspension equilibrated for 60 sec prior to addition of PLA₂. The characteristic lag-burst behaviour of the phospholipase is signalled by a sudden increase in the monomer fluorescence at 380 nm and a subsequent decrease in the eximer fluorescence as shown at the insert on Fig. 15.

Assay conditions for the PLA₂ reaction time profile shown in Fig. 15 were: 0.100 mM unilamellar negatively charged liposomes, 100 µl undiluted cell-free peritoneal fluid, 10 mM HEPES (pH 7.5), 5 mM CaCl₂, and 150 mM NaCl.

## Claims

1. Use of lipid-based drug delivery system for the preparation of a medicament for treatment or prevention of diseases or conditions associated with a localised increase in extracellular PLA₂ activity in cutaneous or subcutaneous tissue of a mammal in the form of a liposome for administration of an active drug substance selected from lysolipid derivatives, said system comprising an edge active compound, and said active drug substance is present in the lipid-based system in the form of a prodrug, said prodrug being a lipid derivative having (a) an aliphatic group of a length of at least 7 carbon atoms and an organic radical having at least 7 carbon atoms, and (b) a hydrophilic moiety, said prodrug furthermore being a substrate for extracellular PLA₂ to the extent that the organic radical can be hydrolytically cleaved off, whereas the aliphatic group remains substantially unaffected, whereby the active drug substance is liberated in the form of a lysolipid derivative which is not a substrate for lysophospholipase.

2. Use according to claim 1, wherein the organic radical which can be hydrolytically cleaved off, is an auxiliary drug substance or an efficiency modifier for the active drug substance.

3. Use according to claim 2, wherein the prodrug is a lipid derivative of the following formula: 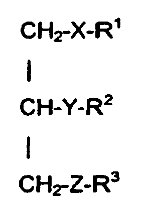 wherein
X and Z independently are selected from O, CH₂, NH, NMe, S, S(O), and S(O)₂;
Y is -OC(O)-, Y then being connected to R² via either the oxygen or carbonyl carbon atom;
R¹ is an aliphatic group of the formula Y¹Y²;
R² is an organic radical having at least 7 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)_{nBr}-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl,
R³ is selected from phosphatidic acid (PO₂-OH), derivatives of phosphatidic acid and bioisosters to phosphatic acid and derivatives thereof.

4. Use according to claim 3, wherein R² is an aliphatic group of a length of at least 7 carbon atoms.

5. Use according to claim 4, wherein R² is a group of the formula Y¹Y².

6. Use according to any of the preceding claims, wherein the prodrug constitutes 15-100 mol% of the total dehydrated lipid-based system.

7. Use according to any of the preceding claims which is in the form of liposomes wherein a second drug substance is incorporated.

8. The use of a lipid based drug delivery system for the preparation of a medicament for treatment or prevention of diseases or conditions associated with a localised increase in extracellular PLA₂ activity in cutaneous or subcutaneous tissue of a mammal in the form of a liposome for administration of an second drug substance, said system comprising an edge active compound, and said second drug substance is incorporated in the system, said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular PLA₂ to the extent that the organic radical can be hydrolytically cleaved off, whereas the aliphatic group remains substantially unaffected, so as to result in an organic acid fragment or an organic alcohol fragment and a lysolipid fragment, said lysolipid fragment not being a substrate for lysophospholipase.

9. Use according to claim 8, wherein the organic radical which can be hydrolytically cleaved off, is an auxiliary drug substance or an efficiency modifier for the second drug substance.

10. Use according to claims 8 - 9, wherein the lipid derivative is a lipid derivative of the following formula: 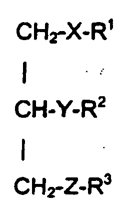 wherein
X and Z independently are selected from O, CH₂, NH, NMe, S, S(O), and S(O)₂;
Y is -OC(O)-, Y then being connected to R² via either the oxygen or carbonyl carbon atom;
R¹ is an aliphatic group of the formula Y¹Y²;
R² is an organic radical having at least 7 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl,
R³ is selected from phosphatidic acid (PO₂-OH), derivatives of phosphatidic acid and bioisosters to phosphatic acid and derivatives thereof.

11. Use according to claim 10, wherein R² is an aliphatic group of a length of at least 7 carbon atoms.

12. Use according to claim 11, wherein R² is a group of the formula Y¹Y².

13. Use according to any of the claims 8-12, wherein the lipid derivative constitutes 15-100 mol% of the total dehydrated system.

14. Use according to any of the claims 7-13, wherein the second drug substance is a therapeutically and/or prophylactically active substance selected from (i) antitumor agents, (ii) antibiotics and antifungals, and (iii) antiinflammatory agents.

15. Use according to any of the preceding claims, wherein the skin diseases or conditions are selected from the group consisting of inflammatory skin conditions, skin cancer and conditions caused by infection of the skin.

16. The use according to claim 15, wherein the inflammatory skin conditions are selected from the group consisting of inflammatory skin diseases such as psoreasis, pityriasis rosea, ichthyosis vulgaris, prurigo nodularis, neurodermatitis, lichen planus, alopecia, erythema multiforme, erythema nodosum, lupus, dermatomyositis, morphoea, pemphigus, pemphigoid, pyoderma gangrenosum, urticaria, atopic dermatitis and eczema.

17. The use according to claims 15 and 16, wherein the increase in extracellular PLA₂ activity is a least 25% compared to the normal level of activity in the relevant part of the skin.

18. The use of the lipid-based delivery system described in any of the preceding claims for the preparation of a topical composition.

19. The use of the lipid-based delivery system described in any of the preceding claims, wherein the topical composition is a cosmetic composition.

## Patentansprüche

1. Verwendung eines auf Lipiden basierenden Arzneistoffabgabesystems für die Zubereitung eines Medikaments für die Behandlung oder die Verhinderung von Krankheiten oder Leiden, die mit einer lokalisierten Erhöhung der extrazellularen PLA₂-Tätigkeit im kutanen oder subkutanen Gewebe eines Säugetiers verbunden sind, in Form eines Liposoms für die Verabreichung eines Arzneiwirkstoffs, ausgewählt aus Lysolipid-Derivaten, wobei das System eine randaktive Verbindung umfasst und der Arzneiwirkstoff im auf Lipiden basierenden System in Form einer Prodrug vorliegt, wobei die Prodrug ein Lipidderivat ist mit (a) einer aliphatischen Gruppe mit einer Länge von zumindest 7 Kohlenstoffatomen und einem organischen Radikal mit zumindest 7 Kohlenstoffatomen und (b) einem hydrophilen Anteil, wobei die Prodrug des Weiteren ein Substrat für extrazellulare PLA₂ dergestalt ist, dass das organische Radikal hydrolytisch abgetrennt werden kann, während die aliphatische Gruppe im Wesentlichen unbeeinträchtigt bleibt, wodurch der Arzneiwirkstoff in Form eines Lysolipid-Derivats freigesetzt wird, das kein Substrat für Lysophospholipase ist.

2. Verwendung nach Anspruch 1, wobei das organische Radikal, welches hydrolytisch abgetrennt werden kann, ein Arzneihilfsstoff oder ein Wirksamkeitsveränderer für den Arzneiwirkstoff ist.

3. Verwendung nach Anspruch 2, wobei die Prodrug ein Lipidderivat mit folgender Formel ist: 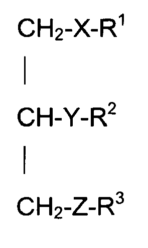 wobei
X und Z unabhängig aus O, CH₂, NH, NMe, S, S(O) und S(O)₂ ausgewählt werden;
Y -OC(O)- ist, Y dann mit R² entweder über das Sauerstoff- oder das Carbonylkohlenstoffatom verbunden wird;
R¹ eine aliphatische Gruppe der Formel Y¹Y² ist;
R² ein organisches Radikal mit zumindest 7 Kohlenstoffatomen ist;
wobei Y¹ -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉ ist und die Summe von n1+2n2+n3+2n4+n5+2n6+n7+2n8+n9 eine Ganzzahl von 9 bis 29 ist; n1 Null oder eine Ganzzahl von 1 bis 29 ist, n3 Null oder eine Ganzzahl von 1 bis 20 ist, n5 Null oder eine Ganzzahl von 1 bis 17 ist, n7 Null oder eine Ganzzahl von 1 bis 14 ist und n9 Null oder eine Ganzzahl von 1 bis 11 ist; und jede von n2, n4, n6 und n8 unabhängig Null oder 1 ist; und Y² CH₃ oder CO₂H ist; wobei jedes Y¹-Y² unabhängig durch Halogen oder C₁₋₄-Alkyl ersetzt werden kann,
R³ ausgewählt wird aus Phosphatidsäure (PO₂-OH), Derivaten von Phosphatidsäure und Bioisosteren zu Phosphatsäure und Derivaten derselben.

4. Verwendung nach Anspruch 3, wobei R² eine aliphatische Gruppe mit einer Länge von zumindest 7 Kohlenstoffatomen ist.

5. Verwendung nach Anspruch 4, wobei R² eine Gruppe der Formel Y¹Y² ist.

6. Verwendung nach einem der vorangehenden Ansprüche, wobei die Prodrug 15 - 100 Mol% des gesamten dehydrierten auf Lipiden basierenden Systems darstellt.

7. Verwendung nach einem der vorangehenden Ansprüche, die in Form von Liposomen erfolgt, wobei ein zweiter Arzneistoff inkorporiert ist.

8. Verwendung eines auf Lipiden basierenden Arzneistoffabgabesystems für die Zubereitung eines Medikaments für die Behandlung oder die Verhinderung von Krankheiten oder Leiden, die mit einer lokalisierten Erhöhung der extrazellularen PLA₂-Tätigkeit im kutanen oder subkutanen Gewebe eines Säugetiers verbunden sind, in Form eines Liposoms für die Verabreichung eines zweiten Arzneistoffes, wobei das System eine grenzaktive Verbindung umfasst, und der zweite Arzneistoff in das System inkorporiert ist und das System Lipidderivate einschließt, die (a) eine aliphatische Gruppe mit einer Länge von zumindest 7 Kohlenstoffatomen und einen organischen Radikal mit zumindest 7 Kohlenstoffatomen und (b) einen hydrophilen Anteil aufweisen, wobei das Lipidderivat des Weiteren ein Substrat für extrazellulare PLA₂ dergestalt ist, dass das organische Radikal hydrolytisch abgetrennt werden kann, während die aliphatische Gruppe im Wesentlichen unbeeinträchtigt bleibt, so dass das Ergebnis ein organisches Säurefragment oder ein organisches Alkoholfragment und ein Lysolipidfragment ist und das Lysolipidfragment kein Substrat für Lysophospholipase ist.

9. Verwendung nach Anspruch 8, wobei das organische Radikal, welches hydrolytisch abgetrennt werden kann, ein Arzneihilfsstoff oder ein Wirksamkeitsveränderer für den zweiten Arzneistoff ist.

10. Verwendung nach den Ansprüchen 8 - 9, wobei das Lipidderivat ein Lipidderivat mit folgender Formel ist: 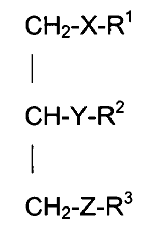 wobei
X und Z unabhängig aus O, CH₂, NH, NMe, S, S(O) und S(O)₂ ausgewählt werden;
Y -OC(O)- ist, Y dann mit R² entweder über das Sauerstoff- oder das Carbonylkohlenstoffatom verbunden wird;
R¹ eine aliphatische Gruppe der Formel Y¹Y² ist;
R² ein organisches Radikal mit zumindest 7 Kohlenstoffatomen ist;
wobei Y¹ -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉ ist und die Summe von n1+2n2+n3+2n4+n5+2n6+n7+2n8+n9 eine Ganzzahl von 9 bis 29 ist; n1 Null oder eine Ganzzahl von 1 bis 29 ist, n3 Null oder eine Ganzzahl von 1 bis 20 ist, n5 Null oder eine Ganzzahl von 1 bis 17 ist, n7 Null oder eine Ganzzahl von 1 bis 14 ist und n9 Null oder eine Ganzzahl von 1 bis 11 ist; und jede von n2, n4, n6 und n8 unabhängig Null oder 1 ist; und Y² CH₃ oder CO₂H ist; wobei jedes Y¹-Y² unabhängig durch Halogen oder C₁₋₄-Alkyl ersetzt werden kann,
R³ ausgewählt wird aus Phosphatidsäure (PO₂-OH), Derivaten von Phosphatidsäure und Bioisosteren zu Phosphatsäure und Derivaten derselben.

11. Verwendung nach Anspruch 10, wobei R² eine aliphatische Gruppe mit einer Länge von zumindest 7 Kohlenstoffatomen ist.

12. Verwendung nach Anspruch 11, wobei R² eine Gruppe der Formel Y¹Y² ist.

13. Verwendung nach einem der Ansprüche 8 - 12, wobei das Lipidderivat 15 - 100 Mol% des gesamten dehydrierten Systems darstellt.

14. Verwendung nach einem der Ansprüche 7 - 13, wobei der zweite Arzneistoff ein therapeutischer und/oder prophylaktischer Wirkstoff ist, ausgewählt aus (i) Antitumormitteln, (ii) Antibiotika und Mitteln gegen Pilzbefall und (iii) entzündungshemmenden Mitteln.

15. Verwendung nach einem der vorangehenden Ansprüche, wobei die Hautkrankheiten oder Hautleiden ausgewählt werden aus der Gruppe, bestehend aus entzündlichen Hautleiden, Hautkrebs und Leiden, hervorgerufen durch eine Infektion der Haut.

16. Verwendung nach Anspruch 15, wobei die entzündlichen Hautleiden ausgewählt werden aus der Gruppe bestehend aus entzündlichen Hautkrankheiten, wie zum Beispiel Psoriasis, Pityriasis rosea, Ichthyosis vulgaris, Prurigo nodularis, Neurodermatitis, Lichen planus, Alopecia, Erythema multiforme, Erythema nodosum, Lupus, Dermatomyositis, Morphoea, Pemphigus, Pemphigoid, Pyoderma gangrenosum, Urticaria, atopische Dermatitis und Ekzeme.

17. Verwendung nach Anspruch 15 und 16, wobei die Erhöhung der extrazellularen PLA₂-Tätigkeit zumindest 25 % im Vergleich zum normalen Grad der Tätigkeit im entsprechenden Teil der Haut ausmacht.

18. Verwendung des in einem der vorangehenden Ansprüche beschriebenen auf Lipiden basierenden Abgabesystems für die Zubereitung einer topischen Zusammensetzung.

19. Verwendung des in einem der vorangehenden Ansprüche beschriebenen auf Lipiden basierenden Abgabesystems, wobei die topische Zusammensetzung eine kosmetische Zusammensetzung ist.

## Revendications

1. Utilisation d'un système de délivrance de médicaments à base de lipide, pour la préparation d'un médicament pour le traitement ou la prévention de maladies ou d'états pathologiques associés à une augmentation localisée de l'activité de la PLA₂ extracellulaire dans le tissu cutané ou sous-cutané d'un mammifère, sous la forme d'un liposome, pour l'administration d'une substance médicamenteuse active, choisie parmi les dérivés de lysolipides, ledit système comprenant un composé actif en termes d'états frontières et ladite substance médicamenteuse active étant présente dans le système à base de lipide sous la forme d'un précurseur de médicament, ledit précurseur de médicament étant un dérivé lipidique ayant (a) un groupe aliphatique d'une longueur d'au moins 7 atomes de carbone et un reste organique ayant au moins 7 atomes de carbone et (b) un fragment hydrophile, ledit précurseur de médicament étant en outre un substrat pour la PLA₂ extracellulaire, si bien que le reste organique peut être éliminé par clivage hydrolytique, tandis que le groupe aliphatique reste substantiellement sous forme non-altérée, la substance médicamenteuse active étant ainsi libérée sous la forme d'un dérivé de lysolipide qui n'est pas un substrat pour une lysophospholipase.

2. Utilisation selon la revendication 1, dans laquelle le reste organique qui peut être éliminé par clivage hydrolytique est une substance médicamenteuse auxiliaire ou un agent modifiant l'efficacité de la substance médicamenteuse active.

3. Utilisation selon la revendication 2, dans laquelle le précurseur de médicament est un dérivé de lipide ayant la formule suivante : 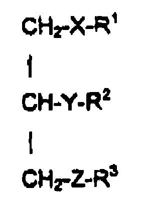 où
X et Z sont choisis, de façon indépendante, parmi O, CH₂, NH, NMe, S, S(O) et S(O)₂ ;
Y est -OC(O)-, Y étant ensuite relié à R² par l'intermédiaire de l'atome d'oxygène ou de l'atome de carbone du carbonyle ;
R¹ est un groupe aliphatique de formule Y¹Y² ;
R² est un reste organique ayant au moins 7 atomes de carbone ;
où Y¹ est -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, et la somme n1+2n2+n3+2n4+n5+2n6+n7+2n8+ n9 est un entier de 9 à 29 ; n1 est égal à zéro ou est un entier de 1 à 29, n3 est égal à zéro ou est un entier de 1 à 20, n5 est égal à zéro ou est un entier de 1 à 17, n7 est égal à zéro ou est un entier de 1 à 14 et n9 est égal à zéro ou est un entier de 1 à 11 ; et chacun des n2, n4, n6 et n8 est, de façon indépendante, égal à zéro ou un ; et Y² est CH₃ ou CO₂H; où chaque Y¹, Y² peut être substitué de façon indépendante par un halogène ou un alkyle en C₁₋₄,
R³ est choisi parmi l'acide phosphatidique (PO₂-OH), les dérivés de l'acide phosphatidique et les bioisostères de l'acide phosphatidique et des dérivés de ceux-ci.

4. Utilisation selon la revendication 3, dans laquelle R² est un groupe aliphatique d'une longueur d'au moins 7 atomes de carbone.

5. Utilisation selon la revendication 4, dans laquelle R² est un groupe de formule Y¹Y².

6. Utilisation selon l'une quelconque des revendications précédentes, dans laquelle le précurseur de médicament représente 15 à 100 % en mole de la totalité du système à base de lipide déshydraté.

7. Utilisation selon l'une quelconque des revendications précédentes, qui est sous la forme de liposomes, dans lesquels une seconde substance médicamenteuse est incorporée.

8. Utilisation d'un système de délivrance de médicaments à base de lipide, pour la préparation d'un médicament pour le traitement ou la prévention de maladies ou d'états pathologiques associés à une augmentation localisée de l'activité de la PLA₂ extracellulaire dans le tissu cutané ou sous-cutané d'un mammifère, sous la forme d'un liposome, pour l'administration d'une seconde substance médicamenteuse active, ledit système comprenant un composé actif en termes d'états frontières et ladite seconde substance médicamenteuse étant incorporée dans le système, ledit système incluant des dérivés lipidiques ayant (a) un groupe aliphatique d'une longueur d'au moins 7 atomes de carbone et un reste organique ayant au moins 7 atomes de carbone et (b) un fragment hydrophile, où le dérivé lipidique est en outre un substrat pour la PLA₂ extracellulaire, si bien que le reste organique peut être éliminé par clivage hydrolytique, tandis que le groupe aliphatique reste substantiellement sous forme non-altérée, ce qui donne un fragment acide organique ou un fragment alcool organique et un fragment lysolipide, ledit fragment lysolipide n'étant pas un substrat pour une lysophospholipase.

9. Utilisation selon la revendication 8, dans laquelle le reste organique qui peut être éliminé par clivage hydrolytique est une substance médicamenteuse auxiliaire ou un agent modifiant l'efficacité de la seconde substance médicamenteuse.

10. Utilisation selon les revendications 8 - 9, dans laquelle le dérivé de lipide est un dérivé de lipide ayant la formule suivante : 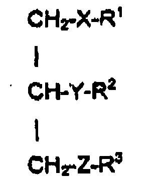 où
X et Z sont choisis, de façon indépendante, parmi O, CH₂, NH, NMe, S, S(O) et S(O)₂ ;
Y est -OC(O)-, Y étant ensuite relié à R² par l'intermédiaire de l'atome d'oxygène ou de l'atome de carbone du carbonyle;
R¹ est un groupe aliphatique de formule Y¹Y² ;
R² est un reste organique ayant au moins 7 atomes de carbone ;
où Y¹ est -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, et la somme n1+2n2+n3+2n4+n5+2n6+n7+2n8+ n9 est un entier de 9 à 29 ; n1 est égal à zéro ou est un entier de 1 à 29, n3 est égal à zéro ou est un entier de 1 à 20, n5 est égal à zéro ou est un entier de 1 à 17, n7 est égal à zéro ou est un entier de 1 à 14 et n9 est égal à zéro ou est un entier de 1 à 11 ; et chacun des n2, n4, n6 et n8 est, de façon indépendante, égal à zéro ou un ; et Y² est CH₃ ou CO₂H ; où chaque Y¹, Y² peut être substitué de façon indépendante par un halogène ou un alkyle en C₁₋₄,
R³ est choisi parmi l'acide phosphatidique (PO₂-OH), les dérivés de l'acide phosphatidique et les bioisostères de l'acide phosphatidique et des dérivés de ceux-ci.

11. Utilisation selon la revendication 10, dans laquelle R² est un groupe aliphatique d'une longueur d'au moins 7 atomes de carbone.

12. Utilisation selon la revendication 11, dans laquelle R² est un groupe de formule Y¹Y².

13. Utilisation selon l'une quelconque des revendications 8 à 12, dans laquelle le dérivé de lipide représente 15 à 100 % en mole de la totalité du système déshydraté.

14. Utilisation selon l'une quelconque des revendications 7 à 13, dans laquelle la seconde substance médicamenteuse est une substance active en termes de traitement et/ou de prophylaxie, choisie parmi (i) les agents antitumoraux, (ii) les antibiotiques et les antifongiques et (iii) les agents antiinflammatoires.

15. Utilisation selon l'une quelconque des revendications précédentes, dans laquelle les états pathologiques cutanés ou les maladies cutanées sont choisies dans le groupe constitué par les états inflammatoires de la peau, le cancer de la peau et les états provoqués par l'infection de la peau.

16. Utilisation selon la revendication 15, dans laquelle les états inflammatoires cutanés sont choisis dans le groupe constitué par les maladies inflammatoires cutanées comme le psoriasis, le pityriasis rosé de Gilbert, l'ichtyose vulgaire, le prurigo nodulaire, la neurodermatose, le lichen plan, l'alopécie, l'érythème polymorphe, l'érythème noueux, le lupus, la dermatomyosite, la morphée, le pemphigus, les affections pemphigoïdes, la pyodermite phagédénique, l'urticaire, la dermite atopique et l'eczéma.

17. Utilisation selon les revendications 15 et 16, dans laquelle l'augmentation de l'activité de la PLA₂ extracellulaire est d'au moins 25 % par rapport au niveau d'activité normal dans la partie de la peau concernée.

18. Utilisation du système de délivrance à base de lipide décrit dans une quelconque des revendications précédentes, pour la préparation d'une composition topique.

19. Utilisation du système de délivrance à base de lipide décrit dans une quelconque des revendications précédentes, dans laquelle la composition topique est une composition cosmétique.
